(19) Europäisches Patentamt
European Patent Office
Office européen des brevets

(11) Numéro de publication: **0 274 453**
**A2**

(12) # DEMANDE DE BREVET EUROPEEN

(21) Numéro de dépôt: **88400010.0**

(22) Date de dépôt: **05.01.88**

(51) Int. Cl.[4]: **C 07 C 103/50**
**A 61 K 31/16**

(30) Priorité: **06.01.87 FR 8700053**

(43) Date de publication de la demande:
**13.07.88 Bulletin 88/28**

(84) Etats contractants désignés:
**AT BE CH DE ES FR GB GR IT LI LU NL SE**

(71) Demandeur: **Société anonyme dite: LABORATOIRE ROGER BELLON**
**159, avenue A. Peretti**
**F-92201 Neuilly-sur-Seine (FR)**

(72) Inventeur: **Cartwright, Terence**
**Le Moulin de l'Etang**
**F-37250 Saint Epain (FR)**

**Bouboutou-Tello, Romaine**
**67-69 Av. A. Briand**
**F-94110 Arcueil (FR)**

**Lelièvre, Yves**
**24, Allée de Venise**
**F-37200 Tours (FR)**

**Fournié-Zaluski, Marie-Claude**
**16, Avenue de Bouvine**
**F-75011 Paris (FR)**

(74) Mandataire: **Portal, Gérard et al**
**Cabinet Beau de Loménie 55, rue d'Amsterdam**
**F-75008 Paris (FR)**

(54) **Nouveaux composés à activité d'inhibiteurs de collagénase, procédé pour les préparer et compositions pharmaceutiques contenant ces composés.**

(57) La présente invention concerne une nouvelle famille de composés chimiques possédant une activité pharmacologique, notamment d'inhibiteur de collagénase, un procédé pour la production de ces composés et des compositions pharmaceutiques les contenant.

Selon l'intention, ces composés répondent à la formule générale

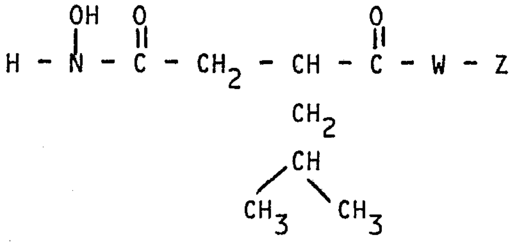

(I)

dans laquelle
-W représente un reste d'acide aminé choisi parmi la valine, la lysine, la norleucine et la méthionine ; et
-Z représente un radical amino ou alcoylamino dont la partie alcoyle qui contient 1 ou 2 atomes de carbone est substituée par un radical phényle ou trifluorophényle,
et comprennent également leurs diastéréoisomères et leurs sels d'addition avec les acides pharmaceutiquement acceptables.

L'intention trouve notamment application dans l'industrie pharmaceutique.

EP 0 274 453 A2

**Description**

Nouveaux composés à activité d'inhibiteurs de collagénase, procédé pour les préparer et compositions pharmaceutiques contenant ces composés.

La présente invention concerne une nouvelle famille de composés chimiques possédant une activité pharmacologique, notamment d'inhibiteur de collagénase, un procédé pour la production de ces composés et des compositions pharmaceutiques les contenant.

Plusieurs types de cellules de mammifères secrètent des métalloprotéases capables de dégrader le collagène interstitiel. Ces collagénases (EC 3.4.23.7) sont considérées comme étant impliquées dans le processus pathologique de plusieurs maladies importantes qui sont caractérisées par la dégradation du collagène telles que les maladies arthritiques et arthrosiques, la maladie périodontale, l'ulcération cornéenne, l'épidermolyse bullosa dystrophique, l'intasion tumorale et la résorption osseuse.

Du fait que la collagénase est l'enzyme qui déclenche l'attaque protéolytique sur les molécules de collagène (qui sont largement résistantes aux autres protéases), des inhibiteurs puissants et spécifiques de la collagénase sont utiles dans le traitement de telles maladies.

La collagénase est une métalloprotéase qui contient du zinc et qui peut être inhibée d'une façon non spécifique par les réactifs chélatant le zinc, tels que l'acide éthylène diamine tétra-acétique (EDTA), l'O-phénanthroline et divers thiols. Il existe certains inhibiteurs naturels, bien que ceux-ci soient des macromolécules qui ne sont probablement pas utilisables directement comme médicaments.

Des inhibiteurs spécifiques ont été conçus pour d'autres métalloprotéases en combinant une capacité de chélation de l'atome de zinc essentiel à l'activité enzymatique avec une structure analogue à celle du substrat enzymatique qui confère la spécificité à l'inhibiteur. Des exemples de ce type d'inhibiteur pour l'enzyme de conversion de l'angiotensine (Angiotensin Converting Enzyme (ACE)) et pour l'enképhalinase sont décrits respectivement dans les brevets européens No. 0 012 401 et 0 054 862, ainsi que dans le brevet européen 0 082 088 qui décrit des dérivés peptidiques de formule générale

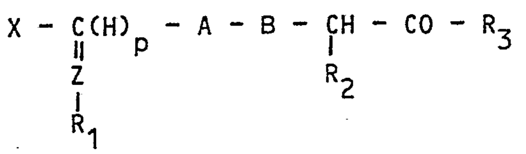

dans laquelle notamment X représente un radical $-CH_2CONHOH$, le radical $>C(H)_p = Z\,R_1$ peut représenter un groupe $>CH-CH_2-C_6H_5$, les radicaux -A-B- peuvent représenter un groupement -CONH-, $R_2$ est un atome d'hydrogène ou un radical alcoyle et $R_3$ est un radical hydroxy ou le reste d'un ester ou d'un amide.

La présente intention concerne des inhibiteurs dérivés des considérations de la spécificité de substrat de la collagénase de mammifères. Ces inhibiteurs sont puissants et sélectifs et n'inhibent pas d'une façon importante l'ACE ou l'enképhalinase.

Il a en effet été trouvé qu'une classe tout-à-fait particulière de dérivés de l'acide hydroxamique définis par la formule générale

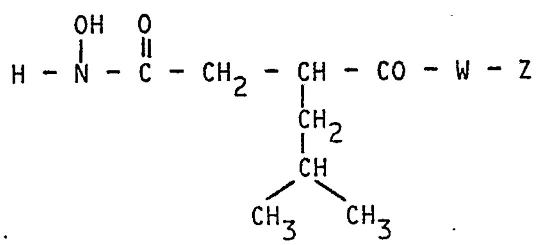

(I)

dans laquelle

W représente un reste d'acide aminé choisi parmi la valine, la lysine, la norleucine ou la méthionine, et Z représente un radical amino ou alcoylamino dont la partie alcoyle qui contient 1 ou 2 atomes de carbone est substituée par un radical phényle ou trifluorométhylphényle, présentait une activité d'inhibiteur de collagénase et était notamment utile pour le traitement de maladies impliquant une destruction excessive du collagène par la collagénase.

La présente intention concerne également les diastéréoisoméres et les sels d'addition d'acides non toxiques pharmaceutiquement acceptables des composés de formule (I).

Plus particulièrement, la présente intention a pour objet des composés de formule I dans laquelle : W représente un groupe L-valyle et Z représente un radical alcoylamino dont la partie alcoyle qui contient 1 ou 2 atomes de carbone est substituée par un radical phényle ou trifluorométhylphényle.

Les composés les plus représentatifs sont les suivants : - Nα -(N-hydroxy isobutyl-2 succinamoyl) L

valinamide

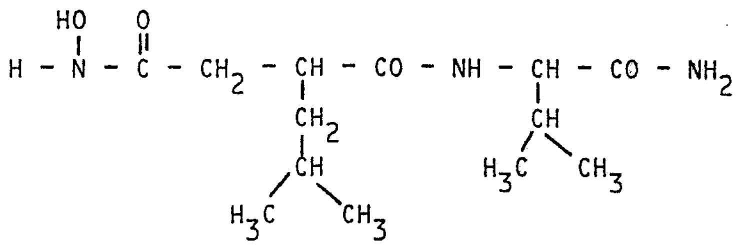

- N-Benzyl N α-(N-hydroxy isobutyl-2 succinamoyl) L valinamide

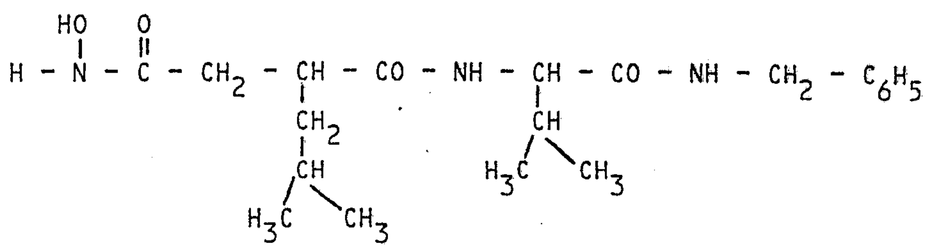

- N -(trifluorométhyl-4 benzyl) N α-(N-hydroxy isobutyl-2 succinamoyl) L valinamide

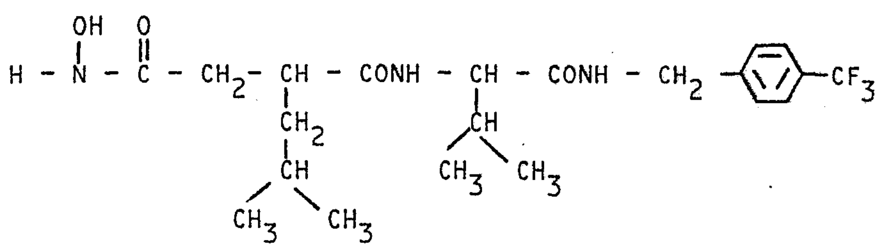

- N-phénéthyl N α-(N-hydroxy isobutyl-2 succinamoyl)L-valinamide

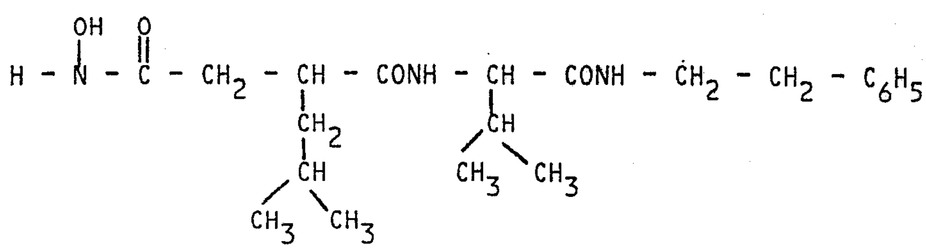

- N-benzyl Nα-(N-hydroxy isobutyl-2 succinamoyl) L norleucine

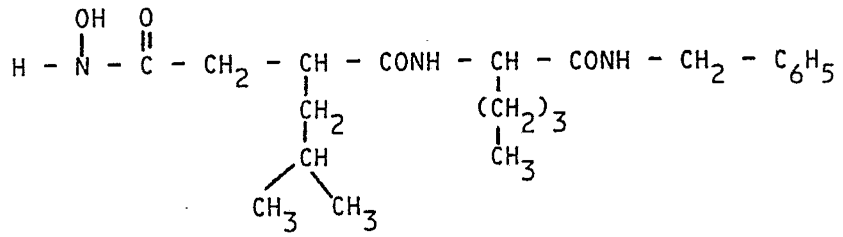

- N-benzyl Nα-(N-hydroxy isobutyl-2 succinamoyl) L lysinamide

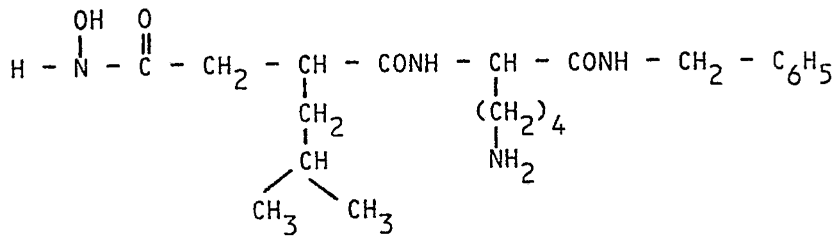

- N-benzyl-Nα(N-hydroxy isobutyl-2 succinamoyl)-L-méthionine amide

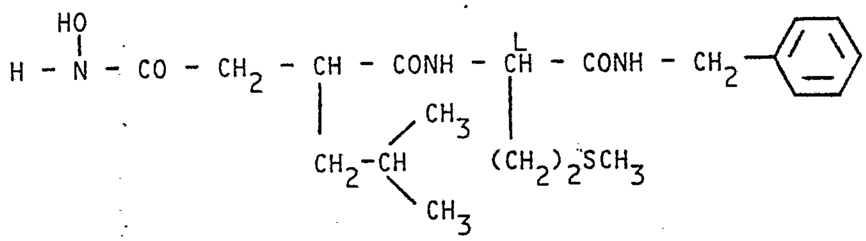

La présente intention concerne également des compositions pharmaceutiques, notamment à activité d'inhibiteur de collagénase, caractérisées en ce qu'elles contiennent, à titre d'ingrédient actif, au moins l'un des composés définis précédemment, en association avec un véhicule ou support non toxique pharmaceutiquement acceptable.

Le procédé général pour la préparation des composés selon la présente intention de formule

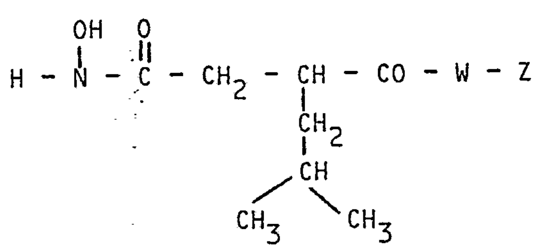

(I)

dans laquelle W et Z sont définis comme ci-dessus, comporte les étapes suivantes :

1) a) la réaction de condensation d'un ester d'acide de formule :

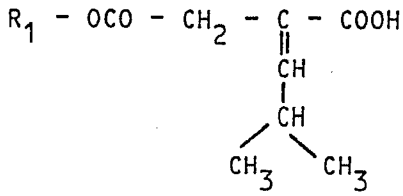

dans laquelle $R_1$ désigne un radical alkyle de 1 à 6 atomes, avec un dérivé d'aminoacide de formule

H - W - Z

dans laquelle W et Z sont définis comme ci-dessus, pour former le composé de formule

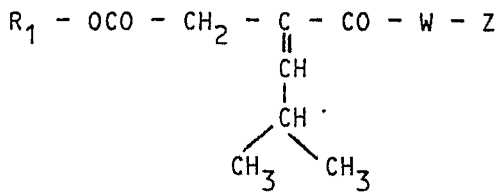

b) l'hydrolyse en milieu alcalin de l'ester formé pour obtenir l'acide correspondant,

c) la condensation de l'acide obtenu avec la O-benzylhydroxylamine pour former le dérivé

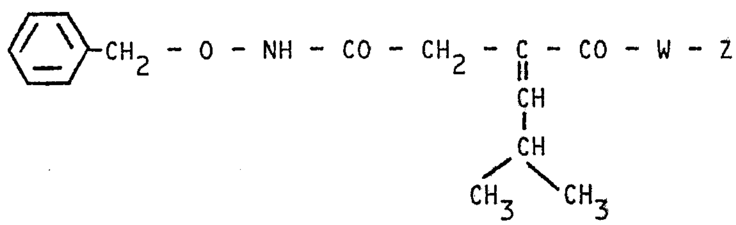

ou bien

a') la réaction de condensation d'un anhydride de formule

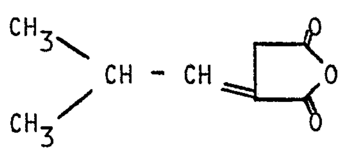

avec la O-benzylhydroxylamine, pour former le dérivé

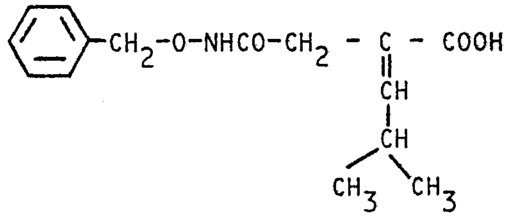

b') la condensation de l'acide obtenu avec un dérivé d'aminoacide de formule générale

H - W - Z

dans laquelle W et Z sont définis comme ci-dessus pour former le dérivé

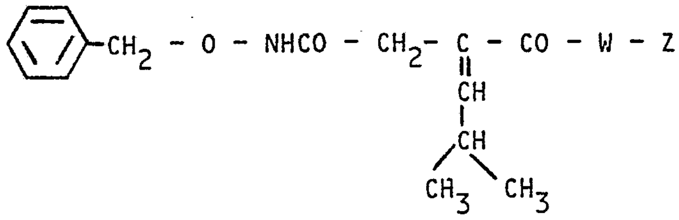

suivies de :

2) l'hydrogénation catalytique du produit obtenu pour obtenir le composé désiré selon la présente intention, ou bien

1') la réaction de condensation d'un anhydride de formule

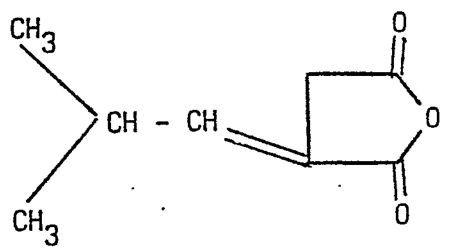

avec la O-tertiobutyloxyhydroxylamine, pour former l'acide N-tertiobutyloxycarbamoyl-3-isobutylidène-2-propanoique de formule

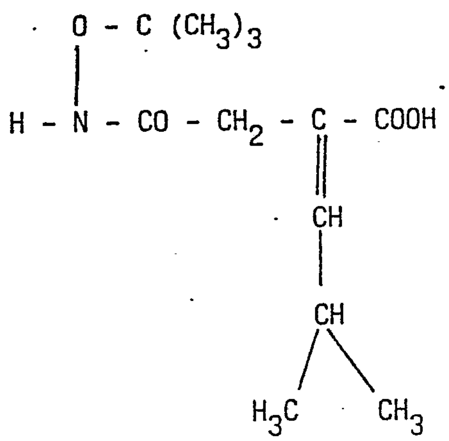

2') l'hydrogénation catalytique de l'acide obtenu en 1') pour former l'acide N-tertiobutyloxycarbamoyl 3-isobutyl-2-propanoïque de formule :

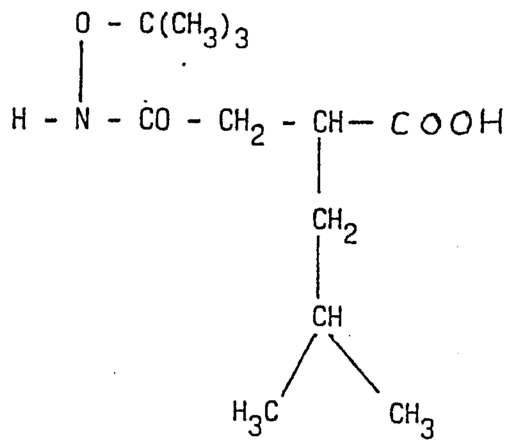

3') la condensation de l'acide obtenu en 2') avec un dérivé d'amino acide de formule générale
H - W - Z
dans laquelle W et Z sont définis comme ci-dessus pour former le dérivé :

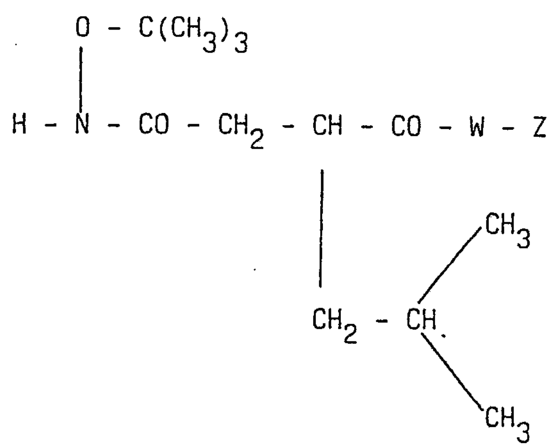

4') La réaction du composé ainsi formé avec un mélange acide trifluoroacétique/trifluoroacétate de bore, pour obtenir le composé désiré de formule I.

Ces composés sont obtenus sous forme de mélange de diastéréoisomères qui peuvent être séparés par des techniques classiques de chromatographie ou par cristallisations fractionnées.

Les esters d'acide de départ, utilisés dans l'étape a) et plus particulièrement l'acide éthoxycarbonyl-3 isobutylidéne-2-propanoïque, sont des produits intermédiaires intéressants pour la synthèse des composés de formule (I).

L'acide éthoxycarbonyl-3-isobutylidène-2-propanoïque peut être préparé par un procédé en quatre étapes :

a) la première étape est une réaction de condensation entre le diester éthylique de l'acide succinique et l'isobutyraldéhyde,

b) la deuxième étape est une réaction d'hydrolyse alcaline du produit de condensation obtenu dans la première étape pour former l'acide isobutylidène succinique ; en effet, la première étape conduit à un mélange complexe dont la purification n'est pas aisée ; ce mélange est donc transformé dans la seconde étape en acide isobutylidène succinique qui est purifié par précipitations ou cristallisations fractionnées,

c) dans la troisième étape, le diacide obtenu subit une déshydratation intramoléculaire pour conduire à un anhydride cyclique,

d) dans la quatrième étape, l'anhydride subit une ouverture de cycle avec estérification sélective pour donner le produit désiré.

Dans les exemples suivants, donnés à titre non limitatif, on appelle forme B la forme isomère qui, après séparation, s'est avérée la forme la plus active comme inhibiteur de collagénase.

Dans le cas où W, dans la formule (I) précitée, est un reste de valine, la forme isomère la plus active est caractérisée en RMN par le déplacement chimique situé à 2 ppm ou en dessous et correspondant au $>CH_{\beta}$ de la valine. La forme A est bien entendu la forme isomère (la moins active) caractérisée en RMN par le

déplacement chimique situé au-dessus de 2 ppm et correspondant au $>CH_\beta$ de la valine.

Exemple 1

-Nα-(N-hydroxy-isobutyl-2-succinamoyl)L-valine

(1) Monoesters éthyliques de l'acide isobutylidène succinique $HOOC-CH_2-C[=CH=CH(CH_3)_2]-COOC_2H_5$ et $H_5C_2OOC-CH_2-C[=CH-CH(CH_3)_2]-COOH$

A 6,1 g (157 mM) de potassium dissous dans 135 ml de terbutanol et portés au reflux, est ajoutée, en 10 min, une solution de 10,25 g (142,4 mM) d'isobutyraldéhyde et de 31 g (178 mM) de succinate de diéthyle fraîchement distillé. Après un chauffage au reflux de 5 min, au mélange refroidi sont additionnés 21 ml de HCl concentré dans 21 g de glace pilée. Le mélange est évaporé et le résidu obtenu est trituré dans l'éther. Le précipité est écarté et la phase éthérée est séchée sur du sulfate de sodium, filtrée et évaporée à sec pour donner un produit huileux (34,5 g ; 97 %), Rf = 0,61 (gel de silice, chloroforme/méthanol/acide acétique 9/1/0,5), constitué par un mélange de monoesters éthyliques de l'acide isobutylidène succinique.

(2) Acide isobutylidène succinique, forme E $HOOC-CH_2-C\ [=CH-CH(CH_3)_2]-COOH$

34,4 g (172 mM) des monoesters précédents (1) sont dissous dans 100 ml d'un mélange éthanol/eau (2/1). A la solution refroidie à 0° C, sont ajoutés 344 ml de soude 2,5N. Après agitation pendant 1 h à 0° C, puis une nuit à +4° C et 2 h à la température ambiante, la solution est filtrée puis évaporée à sec. Le résidu est repris par de l'eau, lavé par l'acétate d'éthyle (4 fois), acidifié par HCl 3N à pH 2-1 et extrait par l'acétate d'éthyle. La solution organique est ensuite lavée par de l'eau, séchée sur du sulfate de sodium, filtrée puis évaporée à sec pour donner une poudre blanche (17,4 g ; 59 %) ; ce composé recristallisé dans l'eau conduit au diacide pur, forme E. Rf = 0,39 (chloroforme/méthanol/acide acétique 9/1/0,5), Point de fusion : 200° C

(3) Anhydride d'acide isobutylidène succinique :

8,2 g (47,9 mM) de diacide (2) sont dissous dans 144 ml d'anhydride acétique. La solution est chauffée au reflux pendant 1 h. Après refroidissement, un précipité est éliminé. La solution est évaporée à sec pour donner un résidu marron contenant de l'anhydride d'acide isobutylidène succinique qui est utilisé tel quel (7,4 g ; 100 %).

(4) Acide éthoxycarbonyl-3-isobutylidéne-2-propanoïque, forme E $H_5C_2OOC-CH_2-C$ $[=CH-CH(CH_3)_2]-COOH$

Une solution de 7,5 g (48,7 mM) de l'anhydride (3) dans 97 ml d'éthanol anhydre est chauffée au reflux pendant 3 h. Après refroidissement, la solution évaporée à sec donne une huile épaisse (8,9 g ; 91 %) qui, cristallisée dans l'éther de pétrole, conduit au produit attendu, l'acide éthoxycarbonyl-3-isobutylidène-2-propanoïque, forme E, sous la forme de cristaux blancs.

Point de fusion 83° C

Rf = 0,56 (chloroforme/méthanol/acide acétique 9/1/0,5)

(5) $H_5C_200C-CH_2-C\ [=CH-CH(CH_3)_2]$-CO-L-valinamide

A une solution de 1 g (5 mM) de l'acide (4) dans le diméthylformamide (DMF) et refroidie à 0° C, sont ajoutées successivement :

- une solution de 777 mg (5 mM) de chlorhydrate de L-valinamide et de triéthylamine (715 µl) dans le DMF,
- une solution de 779 mg (5 mM) d'hydroxybenzotriazole dans le DMF,
- une solution de 1,16 g (5,5 mM) de N,N-dicyclohexylcarbodiimide (DCC) dans le DMF.

Après une heure d'agitation à 0° C, puis environ 20 h à la température ambiante, le précipité de dicyclohexylurée (DCU) est séparé par filtration et le filtrat évaporé à sec. Le résidu obtenu est repris par l'acétate d'éthyle, filtré à nouveau et lavé successivement par $H_2O$ (2 fois), l'acide citrique à 10 % (3 fois), $H_2O$ (1 fois), $NaHCO_3$ à 10 % (3 fois), $H_2O$ (1 fois), une solution saturée de NaCl (1 fois) puis séché sur du sulfate de sodium et évaporé à sec pour donner l'amide ester (5) sous la forme d'une huile épaisse (1,2 g ; 80 %) ; Rf = 0,76 (chloroforme/méthanol 7/3).

(6) $HOOC-CH_2-C\ [=CH-CH)CH_3)_2]$-CO L-valinamide :

A une solution de 1,2 g (3,9 mM) de l'ester éthylique (5) dans 10 ml d'un mélange éthanol/eau (2/1), refroidie à 0° C, sont ajoutés 4,2 ml de soude N. Après 1 h d'agitation à 0° C puis 1 h la température ambiante, le mélange est traité dans les conditions du composé (2) pour donner l'acide (6) sous forme de mousse (1 g ; 78 %).

(7) $C_6H_5-CH_2O-NHCO-CH_2-C\ [=CH-CH(CH_3)_2]$-CO-L-valinamide :

A partir de 749 mg (2,77 mM) de l'acide précédent (6) et de 442 mg (2,77 mM) de chlorhydrate de O-benzylhydroxylamine, on obtient, après traitement dans les conditions décrites pour l'obtention du composé (5), et après chromatographie sur gel de silice (dichlorométhane/acétate d'éthyle), un produit mousseux (394 mg ; 38 %) ;

Rf = 0,23 (acétate d'éthyle).

HO O

(8) H -N-C- $CH_2$-CH [$CH_2$-CH($CH_3$)$_2$]-CO-L-valinamide :

A une suspension de 22 mg de charbon palladié 10 % (100 mg/mM) dans 5 ml de méthanol et de 0,5 ml d'acide acétique, préalablement saturée d'hydrogène, est ajoutée une solution méthanolique de 82 mg (0,22 mM) de dérivé (7). Après agitation pendant 1 h à température ambiante, sous atmosphère d'hydrogène à la pression ordinaire, le catalyseur est éliminé par filtration, le filtrat est évaporé à sec sous vide, pour donner l'hydroxamate : Nα-(N-hydroxy isobutyl-2-succinamoyl)-L-valinamide sous forme d'un mélange de diastéréoisomères (63 mg ; 100 %) ;
Rf = 0,57 et 0,63 (chloroforme/méthanol 7/3).

Exemple 2

N-benzyl-Nα-(N-hydroxy-isobutyl-2-succinamoyl)-L-valinamide

(9) Benzylamide de la t-butyloxycarbonyl-L-valine :

En partant de 1 g (4,6 mM) de t-butyloxycarbonyl-L-valine et de 493 mg (4,6 mM) de benzylamine et en appliquant les conditions de traitement décrites pour l'obtention du composé (5), on obtient le benzylamide de la t-butyloxycarbonyl-L-valine (1,4 g ; 100 %) ;
Rf = 0,73 (chloroforme/méthanol 9/1).

(10) Trifluoroacétate du benzylamide de la L-valine :

1,4 g (4,6 mM) du composé précédent (9) sont solubilisés à 0° C dans 7 ml d'acide trifluoroacétique. Après agitation pendant 30 min à 0° C puis 30 min à la température ambiante, l'excès d'acide est évaporé sous vide et le résidu est lavé plusieurs fois l'éther jusqu'à pH 4 pour donner une poudre blanche (1,4 g 96 %) ;
Rf = 0,51 (chloroforme/méthanol 7/3).

(11) Benzylamide de $H_5C_2OOC$-$CH_2$-C[=CH-CH($CH_3$)$_2$]-CO-L-valine

800 mg (4 mM) de l'acide (4) et 1,28 g (4 mM) du trifluoracétate (10) sont traités dans les conditions d'obtention du composé (5) pour donner l'ester (11) sous la forme d'une huile épaisse (1,54 g ; 99 %) ;
Rf = 0,65 (chloroforme/méthanol 9/1)

(12) Benzylamide de HOOC-$CH_2$-C[=CH-CH($CH_3$)$_2$]-CO-L-valine :

1,47 g (3,78 mM) de l'ester précédent (11) sont traités par 1,62 ml de soude N dans les conditions d'obtention du composé (6) pour conduire à l'acide (12) sous la forme d'une huile (902 mg ; 66 %) ;
Rf = 0,4 (chloroforme/méthanol/acide acétique 9/1/0,5).

(13) Benzylamide de $C_6H_5$-$CH_2$O-NHCO $CH_2$-C[=CH-CH($CH_3$)$_2$]-CO-L-valine

430 mg (1,2 mM) de l'acide (12) et 190 mg (1,2 mM) du chlorhydrate de O-benzylhydroxylamine sont traités dans les conditions d'obtention du composé (7) pour conduire à une mousse qui, après chromatographie sur gel de silice (dichlorométhane/acétate d'éthyle 8/2) donne l'amide (13) (247 mg ; 44 %) ;
Rf = 0,17 (dichlorométhane/acétate d'éthyle 7/3).

HO O

(14) Benzylamide de H -N-C- $CH_2$-CH[$CH_2$-CH($CH_3$)$_2$]-CO-L-valine :

247 mg (0,53 mM) dé l'amide précédent (13) sont hydrogénés dans les conditions d'obtention du composé (8) pour conduire à l'hydroxamate (14) sous forme de paillettes (143 mg ; 72 %) d'un mélange de diastéréoisomères.
Rf = 0,16 et 0,21 (chloroforme/méthanol 9/1) ;
Point de fusion = 145°C ; les deux diastéréoisomères sont ensuite séparés sur gel de silice (chloroforme/méthanol 9/1) en donnant les isoméres purs (14 A) et (14 B), l'isomére B étant le plus actif. RMN 270 (δ H mobiles) : isomère B : 7,87 ppm (NH-Val) ; 8,28 ppm (NH-benzylamide) ; 8,78 ppm (OH) ; 10,45 ppm (NH-O) ; isomère A : 8,08 ppm (NH-Val) ; 8,40 ppm (NH-benzylamide) ; 8,67 ppm (OH) ; 10,39 ppm (NH-O).

Exemple 3

N-benzyl-Nα-(N-hydroxy-isobutyl-2-succinamoyl)-L-valinamide, forme B

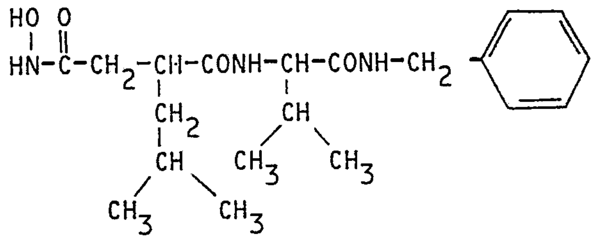

(9) N-benzyl-Nα-tertiobutyloxycarbonyl-L-valinamide

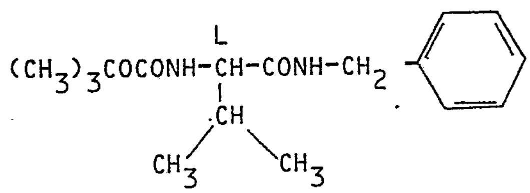

A une solution maintenue en agitation à 0°C de 150 g (0,69 M) de N-tertiobutyloxycarbonyl-L-valine, de 74 g (0,69 M) de benzylamine, de 105,8 g (0,69 M) d'hydroxybenzotriazole hydrate, dans un mélange de 4,5 litres de tétrahydrofuranne et de 0,5 1 de diméthylformamide, on ajoute une solution de 150 g (0,73 M) de dicyclohexylcarbodiimide dans 0,5 1 de diméthylformamide Le mélange réactionnel est agité pendant 1 h à froid puis pendant 20 h vers 20°C. Ensuite, la dicyclohexylurée en suspension est séparée par filtration.

Le filtrat est concentré à sec sous pression réduite (20 mm de mercure, puis 0,5 mm de mercure) à 50°C. Le résidu est repris par 3 l d'acétate d'éthyle et la suspension est filtrée. Le filtrat est lavé successivement :

- 1 fois par 0,6 l de solution aqueuse d'acide citrique à 4 % p/v,
- 2 fois par 1,2 l au total de solution aqueuse de bicarbonate de sodium à 4 % p/v,
- 5 fois par 2 l au total de solution aqueuse de chlorure de sodium à 250 g/l,

La phase organique, séchée sur sulfate de sodium concentrée à sec sous pression réduite (20 mm de mercure) à 50°C donne 213 g (0,69 M) du composé (9) N-benzyl-Nα-tertiobutyloxycarbonyl-Lvalinamide sous la forme d'un solide blanc.

Rf = 0,57, Silicagel ; chloroforme-méthanol (98/2 en volumes).

(10) N-benzyl-L-valinamide

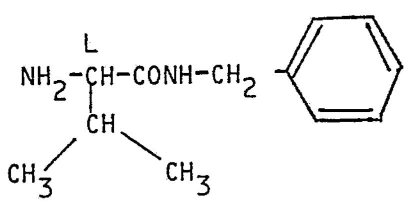

214 g (0,7 M) du composé précédent (9) sont solubilisés à 0°C dans 650 cm$^3$ d'acide trifluoroacétique. Après agitation pendant 30 min à 0°C puis 30 min à 20°C, la solution est concentrée à sec sous pression réduite (1 mm de mercure) à 35°C.

Le résidu huileux peu fluide obtenu, repris par 300 cm$^3$ d'hexane est à nouveau concentré à sec (20 mm de mercure, puis 1 mm de mercure) à 35°C.

Le résidu dissous dans 2,5 1 d'acétate d'éthyle donne une solution qui est lavée successivement :

- 2 fois par 2,6 l au total d'une solution aqueuse de carbonate de sodium (à 8 % p/v),
- 1 fois par 0,5 l d'eau distillée,
- 3 fois par 1,2 l au total de solution aqueuse de chlorure de sodium (à 250 g/l).

La phase acétate d'éthyle, séchée sur sulfate de sodium, concentrée à sec sous pression réduite (20 mm de mercure, puis 1 mm de mercure) à 45°C donne 183 g de N-benzyl-L-valinamide (encore partiellement salifié) sous la forme d'une huile gommeuse qui est utilisée telle quelle lors de l'étape de synthèse suivante.

(11) N-benzyl-Nα-(éthoxycarbonyl-3-isobutylidène-2-propanoyl)-Lvalinamide

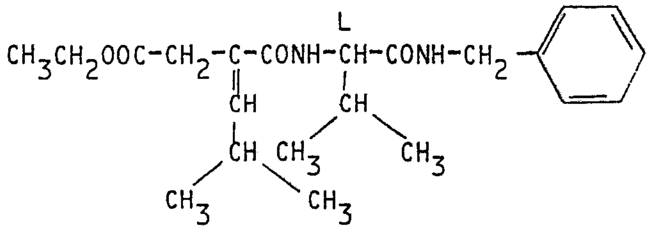

A une solution de 139 g (0,7 M) de l'acide carbéthoxy-3-isobutylidène-2-propanoïque dans 4 1 de tétrahydrofuranne, sont ajoutés successivement :

- une solution de 184 g (0,7 M théorique) du composé (10) dans 2 l de tétrahydrofuranne,
- 97 $cm^3$ de triéthylamine (0,7 M)
- 107 g (0,7 M) d'hydroxybenzotriazole hydrate.

Le mélange agité ést refroidi à 0°C environ puis est additionné d'une solution de 151 g (0,73 M) de dicyclohexylcarbodiimide dans 1,1 l de chloroforme.

Après 1 h d'agitation à 0°C puis 20 h à une température voisine de 20°C, le précipité de dicyclohexylurée est séparé par filtration et le filtrat est concentré à sec sous pression réduite (20 mm de mercure) à 45°C.

Le résidu est repris par 1,5 l d'acétate d'éthyle puis filtré pour éliminer le précipité de dicyclohexylurée.

Le filtrat est lavé successivement :

- 3 fois par 2,1 l au total de solution aqueuse de bicarbonate de sodium (à 8 % p/v),
- 2 fois par 1 l au total de solution aqueuse d'acide citrique (à 4 % p/v)
- 1 fois par le mélange de 20 $cm^3$ de solution aqueuse de bicarbonate de sodium (à 10 % p/v) et de 200 $cm^3$ de solution aqueuse de chlorure de sodium (à 250 g/l),
- 4 fois par 1,6 l au total de solution aqueuse de chlorure de sodium (à 250 g/l).

La phase acétate d'éthyle, séchée sur sulfate de sodium, puis concentré à sec sous pression réduite (20 mm de mercure) à 5°C donne un solide qui donne après agitation dans 10 l d'éther de pétrole pendant 5 h, après séparation par filtration et séchage à l'air, 179 g (0,46 M) du composé (11),N-benzyl-Nα-(éthoxycarbonyl3-isobutylidène-2-propanoyl)-L-valinamide, sous la forme d'un solide blanchâtre.

Rf = 0,56, Silicagel ; acétate d'éthyle-cyclohexane (1/1 en volumes).

Point de fusion (Kofler) = 110°C

Rendement : 66 %.

(12) N-benzyl-Nα-(carboxy-3-isobutylidène-2-propanoyl)-L-valin-amide

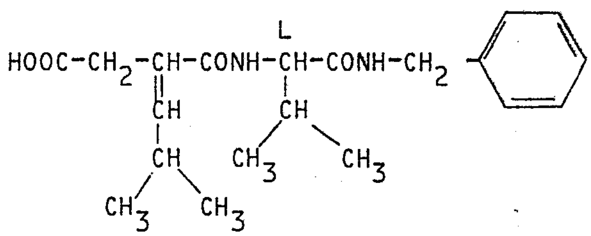

A une solution agitée, refroidie à une température voisine de 0°C de 34,7 g (89 mM) du composé précédent (11) dans 140 $cm^3$ d'éthanol, sont ajoutés goutte à goutte en 30 min 98 $cm^3$ d'une solution de soude 1N.

Après 1 h d'agitation à 0°C puis 2 h à une température voisine de 20°C, le mélange réactionnel est neutralisé à 0°C par de l'acide chlorhydrique 1N. L'éthanol est chassé par évaporation sous pression réduite (20 mm de mercure) à 50°C.

Le résidu aqueux, acidifié à pH 2 par de l'acide chlorhydrique 1N est extrait 2 fois par 1 l au total d'acétate d'éthyle.

La phase organique, lavée successivement :

- 1 fois par 200 $cm^3$ d'eau distillée,
- 3 fois par 900 $cm^3$ au total de solution aqueuse de chlorure de sodium (à 250 g/l) puis séchée sur sulfate de sodium et concentrée à sec sous pression réduite (20 mm de mercure) à 45°C donne 30 g (83 mM) du composé (12) sous la forme d'une meringue blanchâtre.

Rf = 0,22, Silicagel ; chloroforme-méthanol (96/4 en volumes)

Rendement : 93 %.

(13) N-benzyl-Nα-(N-benzyloxycarbamoyl-3-isobutylidène-2-propanoyl)-valinamide

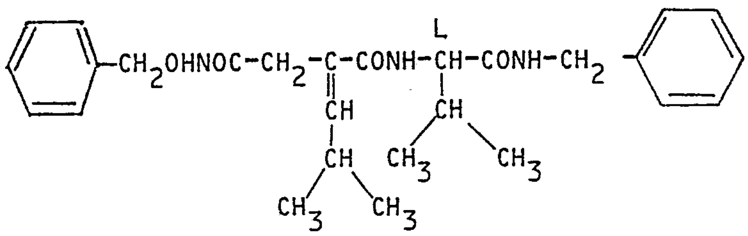

A une solution de 15,7 g (43,6 mM) du composé précédent (12) dans 100 $cm^3$ de diméthylformamide, à 0° C, sont ajoutés successivement, en agitant :

- une solution de 6,95 g (43,6 mM) de chlorhydrate de O-benzylhydroxylamine dans 150 $cm^3$ de diméthylformamide,
- 6,1 $cm^3$ (43,6 mM) de triéthylamine,
- une solution de 6,68 g (43,6 mM) d'hydroxybenzotriazole-hydrate dans 100 $cm^3$ de diméthylformamide,
- une solution de 18,5 g (43,6 mM) de p-toluènesulfonate de [(méthyl- 4-morpholino)- 2-éthyl]--3-cyclohexyl 1-carbodiimide dans 100 $cm^3$ de diméthylformamide.

Après 1 h à 0° C puis 20 h à une température voisine de 20° C, le mélange réactionnel est concentré à sec sous pression réduite (1 mm de mercure) à 45° C.

Le résidu, dissous dans 1 l de chloroforme est lavé successivement :

- 1 fois par 200 $cm^3$ d'eau distillée,
- 1 fois par 400 $cm^3$ de solution aqueuse de bicarbonate de sodium (à 4 % p/v),
- 1 fois par 300 $cm^3$ de solution aqueuse de bicarbonate de sodium (à 0,4 % p/v),
- 3 fois par 600 $cm^3$ au total d'eau distillée.

La phase chloroformique, séchée par sulfate de sodium est concentré à sec sous pression réduite (20 mm de mercure) à 45° C.

L'huile obtenue, agitée pendant 20 h dans 300 $cm^3$ d'éther donne une poudre blanche que l'on sépare par filtration et séche à l'air.

On obtient ainsi 7,3 g (15,7 mM) du composé (13) N-benzyl-Nα-(N-benzyloxycarbamoyl-3-isobutylidène-2-propanoyl)-Lvalinamide sous la forme d'un solide blanc.

Rf = 0,51, Silicagel, chloroforme-méthanol (90/10 en volumes)
Point de fusion (Kofler) = 159° C
Rendement : 36 %.

(14) N-benzyl-Nα-(N-hydroxy-isobutyl-2-succinamoyl)-L-valinamide, forme B

A une suspension de 6,7 g de palladium sur noir (à 10 % de palladium) dans 2,7 l d'acide acétique et 500 $cm^3$ de méthanol, préalablement saturée d'hydrogéne, est ajoutée une solution de 6,7 g (14,4 mM) du composé (13) dans 500 $cm^3$ de méthanol.

Après agitation pendant 4 h sous atmosphère d'hydrogène à température ordinaire, la suspension réactionnelle est purgée 30 min par un courant d'azote.

Le catalyseur est éliminé par filtration, le filtrat est concentré à sec sous pression réduite (20 mm de mercure puis 1 mm de mercure) à 45° C.

Le résidu trituré dans 200 $cm^3$ d'éther donne une poudre qui, séparée par filtration, est ensuite solubilisée dans 50 $cm^3$ de mélange chloroforme-méthanol (95/5 en volumes).

La solution obtenue est chargée sur une colonne de 6,7 cm de diamétre contenant 1200 g de silice neutre (0,04-0,063 mm) puis est éluée avec 12 l de mélange chloroforme-méthanol (95/5 en volumes) en recueillant des fractions de 100 $cm^3$.

Les fractions 95 à 120 réunies et concentrées à sec sous pression réduite (20 mm de mercure) à 45° C donnent 0,46 g (1,22 mM) du composé (14) N-benzyl-Nα-(N-hydroxy-isobutyl-2-succinamoyl)-L-valinamide, forme B, sous la forme d'un solide beige clair. Rf = 0,38, Silicagel ; chloroforme-méthanol (85/15 en volumes)
Point de fusion = 192° C.
RMN DMSO $dd_6$ (400 MHz, δ en ppm, J en Hz)
10.35, bs, 1H, OH ou NH
8.7, s, 1H, NH ou OH
8.3, t, 1H, $\underline{NH}CH_2$
7.9, d, 1H, $\underline{NH}$-CH
7.20 à 7,30, $\underline{m}$, 5 H, $C_6H_5$
4.20, ABX, 2H, $\underline{CH_2}NH$
4.10, dd, (J = 8 $\underline{et}$ 8), 1H, CHα
2,75, bm, 1H, CHCO
2 et 2.15, ABX, 2H, $\underline{CH_2}CO$

2. , m, 1H, CH isopropyle
1,45, m, 1H, CH isopropyle
1.1 et 1.45, ABXX', 2H, $C\underline{H_2}CH$
0.8, m, 12H, $4xCH_3$ isopropyle

Example 4

(15) N-(trifluorométhyl-4-benzyl)-Nα-tertiobutyloxycarbamoyl-L-valinamide

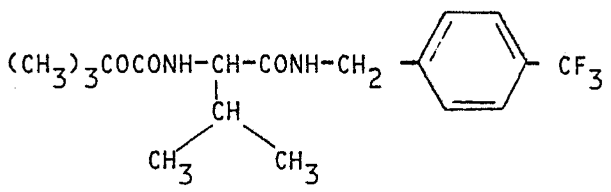

A une solution maintenue sous agitation à +5°C de 5 g (23 mM) de N-tertiobutyloxycarbonyl-L-valine, de 4,02 g (23 mM) de trifluorométhyl-4-benzylamine, de 3,53 g (23 mM) d'hydroxybenzotriazole-hydrate, dans un mélange de 90 $cm^3$ de tétrahydrofuranne et de15 $cm^3$ de diméthylformamide, on ajoute une solution de 4,98 g (24 mM) de dicyclohexylcarbodiimide dans 30 $cm^3$ de chloroforme.

Le mélange réactionnel est agité pendant 1 h vers +5°C puis pendant 20 h vers 20°C. Ensuite, la dicyclohexylurée en suspension est séparée par filtration.

Le filtrat est conçentré à sec sous pression réduite (20 mm de mercure puis 1 mm de mercure) à 50°C. On obtient ainsi un résidu huileux que l'on dissout dans 300 $cm^3$ de chloroforme.

Cette solution est lavée successivement :
- 2 fois par 200 $cm^3$ au total de solution aqueuse de bicarbonate de sodium à 2 % p/v,
- 1 fois par 70 $cm^3$ d'eau distillée,
- 1 fois par 100 $cm^3$ de solution aqueuse d'acide citrique à 2 % p/v
- puis sans attendre, 1 fois par 50 $cm^3$ de solution aqueuse de bicarbonate de sodium à 0,05 % p/v,
- enfin par 3 fois 180 $cm^3$ au total d'eau distillée.

La phase chloroformique, séchée sur sulfate de sodium, est concentrée à sec sous pression réduite (20 mm de mercure) à 40°C.

Au résidu huileux sont ajoutés 200 $cm^3$ d'éther éthylique. La dicyclohexylurée en suspension est séparée par filtration et le filtrat est à nouveau concentré comme précédemment, 40°C.

Le solide blanc obtenu est trituré dans 100 $cm^3$ d'éther de pétrole. La suspension est filtrée, le solide est séché à l'air.

On obtient ainsi 7,9 g du composé (15) (21 mM) N-(trifluorométhyl-4-benzyl) -Nα -tertiobutyloxycarbamoyl -L -valinamide, sous la forme d'un solide blanc.
Rf = 0,6, Silicagel ; chloroforme-méthanol (95/5 en volumes)
Point de fusion (Kofler) = 124-126°C
Rendement 92 %

(16) N-(trifluorométhyl-4-benzyl)-L-valinamide,trifluoroacétate

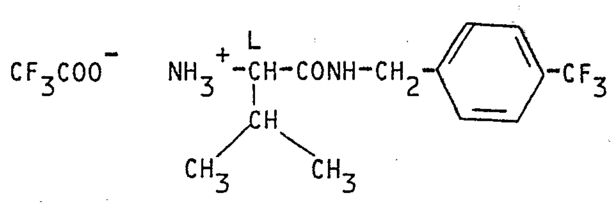

7,9 g (21 mM) du composé précédent (15) sont solubilisés à 0°C dans un mélange de 40 $cm^3$ de dichlorométhane et de 25 $cm^3$ d'acide trifluoroacétique.

Après agitation pendant 1 h à 30°C, on chasse le dichlorométhane sous pression réduite (20 mm de mercure) à 0°C, puis l'excés d'acide trifluoroacétique (1 mm de mercure) à 30°C.

Le résidu huileux obtenu est lavé 4 fois par 120 $cm^3$ au total d'éther de pétrole.

Le résidu gommeux est dissous dans 100 $cm^3$ d'éther puis concentré à sec sous pression réduite (20 mm de mercure).

Le résidu pateux, après 20 h à une température voisine de 20°C dans le mélange de 200 $cm^3$ d'éther de

pétrole et de 30 $cm^3$ d'éther donne une poudre blanche que l'on sépare par filtration.

Le solide est ensuite lavé par 20 $cm^3$ d'éther de pétrole et séché sous pression réduite (20 mm de mercure) à 20°C donnant ainsi 7,24 g du composé (16) (18,4 mM) N-(trifluorométhyl-4-benzyl)-L-valinamide,trifluoroacétate sous la forme d'un solide blanc.

Point de fusion (Kofler) = 145-148°C

Rf ≅ 0,3, Silicagel ; chloroforme-méthanol (80/20 en volumes)

Rendement = 82 %.

(17) N-(trifluorométhyl-4-benzyl)-Nα-(éthoxycarbonyl-3-isobutylidène-2-propanoyl)-L-valinamide

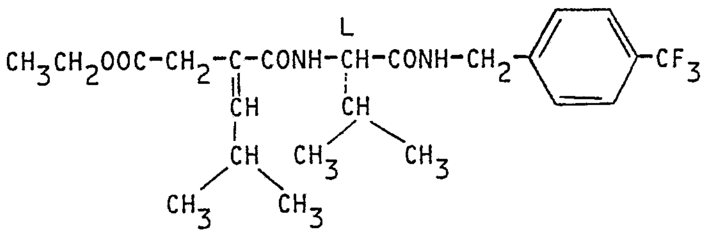

A une solution de 3,69 g (18,4 mM) de l'acide carbéthoxy 3-isobutylidène-2-propanoïque dans 40 $cm^3$ de tétrahydrofuranne, sont ajoutés successivement :

- une solution de 7,24 g (18,4 mM) du composé (16) et 2,58 $cm^3$ de triéthylamine dans 70 $cm^3$ de chloroforme,
- une solution de 2,82 g (18,4 mM) d'hydroxybenzotriazole hydrate dans 50 $cm^3$ de tétrahydrofuranne.

Le mélange agité est refroidi à 0°C puis est additionné d'une solution de 3,98 g (19,3 mM) de dicyclohexylcarbodiimide dans 40 $cm^3$ de chloroforme.

Après 1 h d'agitation à 0°C, puis environ 20 h à une température voisine de 20°C, le précipité de dicyclohexylurée est séparé par filtration et le filtrat est concentré à sec sous pression réduite (20 mm de mercure) à 40°C.

Le résidu est dissous dans 300 $cm^3$ de chloroforme, la solution est lavée successivement :

- 2 fois par 120 $cm^3$ au total de solution aqueuse de bicarbonate de sodium à 2 % p/v,
- 1 fois par 50 $cm^3$ d'eau distillée,
- 1 fois par 50 $cm^3$ de solution aqueuse d'acide citrique à 4 % p/v,
- 2 fois par 100 $cm^3$ au total d'eau distillée.

La phase chloroformique séchée sur sulfate de sodium est concentrée à sec sous pression réduite (20 mm de mercure) à 40°C. Le résidu huileux est repris par un mélange de 200 $cm^3$ d'éther et de 100 $cm^3$ d'éther de pétrole.

Le précipité de dicyclohexylurée est séparé par filtration. Le filtrat, concentré à sec sous pression réduite (20 mm de mercure) à 40°C donne 8,1 g d'une huile jaunâtre que l'on dissout dans un mélange de 50 $cm^3$ d'acétate d'éthyle-cyclohexane (30/70 en volumes).

Cette solution est chargée sur une colonne de 5 cm de diamétre contenant 500 g de silice neutre (0,04-0,063 mm), puis éluée successivement par :

- 2,6 l d'un mélange acétate d'éthyle-cyclohexane (30/70 en volumes),
- 2,0 l d'un mélange acétate d'éthyle-cyclohexane (50/50 en volumes), en recueillant des fractions de 65 $cm^3$.

Les fractions 48 à 74 réunies concentrées à sec sous pression réduite (20 mm de mercure) à 50°C donnent 7,15 g (15,6 mM) du composé (17), N-(trifluorométhyl-4-benzyl)-Nα-(éthoxycarbonyl-3-isobutylidène-2-propanoyl)-L-valinamide, sous la forme de cristaux blancs.

Rf= 0,3, Silicagel ; acétate d'éthyle-cyclohexane (30/70 en volumes)

Point de fusion (Kofler) = 98-100°C

Rendement 85 %

(18) N-(trifluorométhyl-4-benzyl)-Nα-(carboxy-3-isobutylidène-2-propanoyl)-L-valinamide

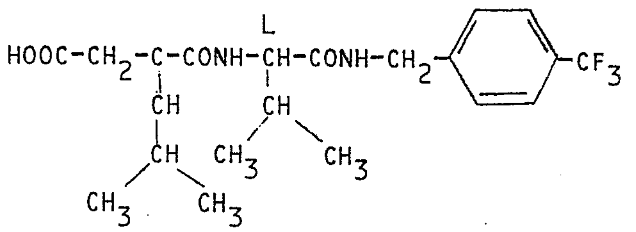

A une solution agitée, refroidie à +5° C environ, de 7,1 g (15,5 mM) du composé précédent (17) dans 35 $cm^3$ d'éthanol, sont ajoutés goutte à goutte en 15 min 17 $cm^3$ d'une solution d'hydroxyde de sodium 1N.

Après 30 minutes vers 0° C puis 2 h à une température voisine de 20° C, le milieu réactionnel est filtré et le filtrat est concentré à 20 $cm^3$ sous pression réduite (1 mm de mercure) à 20° C.

Le résidu huileux, repris par 80 $cm^3$ d'eau distillée, est lavé 2 fois par 140 $cm^3$ au total d'éther.

La phase aqueuse acidifiée à pH ≅ 2 par de l'acide chlorhydrique 4N est extraite par 300 $cm^3$ d'acétate d'éthyle.

La phase acétate d'éthyle, lavée par 50 $cm^3$ de solution aqueuse de chlorure de sodium (à 250 g/L), séchée sur sulfate de sodium puis concentrée à sec sous pression réduite (20 mm de mercure) à 50° C donne 5,78 g (13,5 mM) du composé (18) N-(trifluorométhyl-4-benzyl)-Nα-(carboxy-3-isobutylidène-2-propanoyl) -L-valinamide, sous la forme d'une meringue blanche.

Rf = 0,25, Silicagel ; chloroforme-méthanol (90/10 en volumes)
Rendement = 87 %.

(19) <u>N-(trifluorométhyl-4-benzyl)-Nα-(N-benzyloxycarbamoyl-3-isobutylidéne-2-propanoyl)-L-valinamide</u>

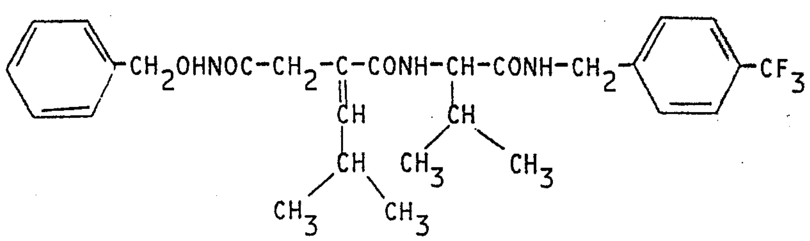

A une solution de 5 g (11,68 mM) du composé (18), dans 50 $cm^3$ de diméthylformamide, sont ajoutés successivement :

- une solution de 1,86 g (11,68 mM) de chlorhydrate de O-benzylhydroxylamine dans 100 $cm^3$ de diméthylformamide
- 1,63 $cm^3$ (11,68 mM) de triéthylamine,
- une solution de 1,79 g (11,68 mM) d'hydroxybenzotriazole hydrate dans 50 $cm^3$ de diméthylformamide.

Le mélange agité est additionné en 2 min d'une solution de 4,95 g (11,68 mM) de p-toluènesulfonate de [(méthyl-4-morpholino)-2-éthyl]-3-cyclohexyl-1-carbodiimide dans 60 $cm^3$ de diméthylformamide.

Après 24 h d'agitation à une température voisine de 20° C, le mélange réactionnel est concentré à sec sous pression réduite (1 mm de mercure) à 50° C.

Le résidu est dissous dans 400 $cm^3$ de chloroforme, la solution est lavée successivement :

- 2 fois par 200 $cm^3$ au total d'eau distillée,
- 2 fois par 200 $cm^3$ au total de solution aqueuse de bicarbonate de sodium (à 4 % p/v),
- 2 fois par 240 $cm^3$ au total de solution aqueuse d'acide citrique (à 4 % p/v),
- 4 fois par 400 $cm^3$ au total d'eau distillée.

La phase chloroformique, séchée sur sulfate de sodium est concentrée à sec sous pression réduite (20 mm de mercure) à 50° C. Le solide blanc résultant, cristallisé dans 80 $cm^3$ d'acétonitrile est séparé par filtration puis séché sous pression réduite (20 mm de mercure) à 20° C.

On obtient 2,12 g (3,97 mM) du composé (19) N-(trifluorométhyl-4-benzyl)-Nα (N-benzyloxy-carbamoyl-3-isobutylidène-2-propanoyl)-L-valinamide, sous la forme d'un solide blanc.

Rf = 0,37, Silicagel ; chloroforme-méthanol (97/3 en volumes) Point de fusion (Kofler) = 170° C
Rendement = 34 %.

(20) <u>N-(trifluorométhyl-4-benzyl)-Nα-(N-hydroxy-isobutyl-2-succinamoyl)-L-valinamide, forme B</u>

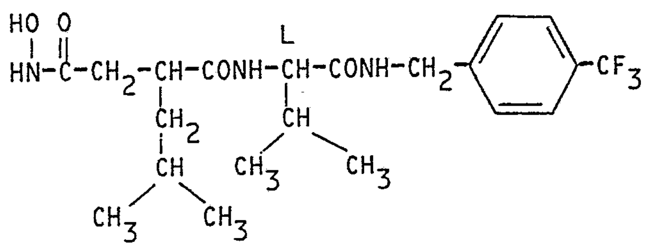

A une suspension de 2,1 g de palladium sur noir (à 10 % de palladium) dans 30 $cm^3$ de µéthanol et 2,5 $cm^3$ d'acide acétique, préalablement saturée d'hydrogéne, est ajoutée une solution de 2,1 g (3,94 mM) du composé (19) dans 30 $cm^3$ de méthanol et 2,5 $cm^3$ d'acide acétique.

Après agitation sous atmosphère d'hydrogène endant 4 h, à une température voisine de 20°C, la suspension réactionnelle est purgée 30 min par un courant d'azote.

Le catalyseur est éliminé par filtration, le filtrat est concentré à sec sous pression réduite (20 mm de mercure) à 40°C.

Le résidu trituré dans 100 $cm^3$ d'éther donne une poudre qui, séparée par filtration, est ensuite solubilisée dans 30 $cm^3$ de méthanol.

Cette solution additionnée de 2 g de silice neutre (0,04-0,063 mm) est concentrée à sec sous pression réduite (20 mm de mercure) à 40°C.

Le solide obtenu, mis en suspension dans 20 $cm^3$ d'un mélange chloroforme-méthanol (90/10 en volumes) est chargé sur une colonne de 3,6 cm de diamétre contenant 150 g de silice neutre (0,04-0,063 mm) puis est élué avec 750 $cm^3$ de mélange chloroformeméthanol (90/10 en volumes) en recueillant des fractions de 15 $cm^3$.

Les fractions 39 à 48 réunies, sont concentrées à sec sous pression réduite (20 mm de mercure) à 50°C

Le résidu, trituré dans 50 $cm^3$ d'éther, donne une poudre qui, séparée par filtration, séchée sous pression réduite (20 mm de mercure) 20°C conduit à l'obtention de 0,16 g (0,36 mM) du composé (20) N-(trifluorométhyl-4-benzyl)-Nα-(N-hydroxyisobutyl-2succinamoyl)-L-valinamide, forme B.

Rf = 0,29,Silicagel chloroforme-méthanol (90/10 en volumes)

Point de fusion (Kofler) = 228°C (peu net)

Spectre de RMN (DMSO $d_6$ 250 Mhz, δ en ppm, J en Hz)

10.4, bs, 1H, NH ou OH
8.70, bs, 1H, OH ou NH
8.50, t, 1H, NH-$CH_2$
7.95, d, (J=8,5), 1H, NHCH
7.45 et 7.65 AB, (J=8), 4H, $C_6H_4$
4.35, bm, ABX limite, 2H, $CH_2N$
4.10, dd, (J=8,5 et 8), 1H, CHX
2.80, m, 1H, CHCO
2.15 et 2, ABX, 2H, $CH_2CO$
2, m, 1H, CH isopropyle
1.4, m, 1H, CH isopropyle
1.4 et 1.05, 2m, 2H, $CH_2$
0.7 à 0.9, 4d, 4x 3H, 4 Me

Exemple 5

(21) N-phénéthyl-Nα-tertiobutyloxycarbonyl-L-valinamide

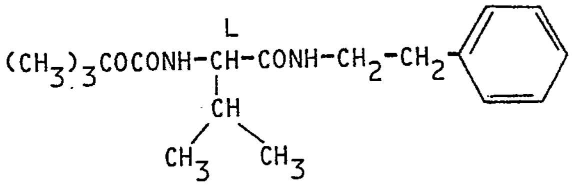

En partant de 5 g (23 mM) de N-tertiobutyloxycarbonyl-L-valine et de 2,7 g (23 mM) de phényl-2-éthylamine et en appliquant les conditions de traitement décrites dans l'obtention du composé (15), on obtient 7,3 g (22,8 mM) du composé (21), N-phénéthyl-Nα- tertiobutyloxycarbonyl-L-valinamide, sous la forme d'un solide blanc.

Rf = 0,6 Silicagel chloroforme-méthanol (95/5 en volumes).

Point de fusion (Kofler) = 119°C
Rendement ≃ 100 %.

(22) N-phénéthyl-L-valinamide,trifluoroacétate

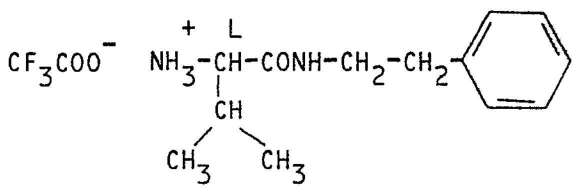

7,3 g (22,8 mM) du composé précédent (21) sont dissous 0°C dans un mélange de 40 cm$^3$ de dichlorométhane et de 2,5 cm$^3$ d'acide trifluoroacétique.

Après agitation pendant 30 min à 0°C puis 1 h à une température voisine de 20°C, on chasse le dichlorométhane sous pression réduite (20 mm de mercure) à 30°C puis l'excés d'acide trifluoroacétique (1 mm de mercure) à 30°C.

Le résidu huileux, lavé 5 fois par 1,5 l au total d' éther de pétrole, donne une suspension blanche.

Le solide, séparé par filtration, est ensuite recristallisé dans 150 cm$^3$ d'éther puis séché sous pression réduite (20 mm de mercure) à 20°C.

On obtient 6,0 g (18 mM) du composé (22) N-phénéthyl-L-valinamide,trifluoroacétate, sous la forme de cristaux blancs. Rf = 0,40 Silicagel, chloroforme-méthanol (80/20 en volumes).
Point de fusion (Kofler) = 124°C.
Rendement = 80 %.

(23)N-phénéthyl-Nα-(éthloxycarbonyl-3-isobutlidène-2-propanoyl)-L-valinamide

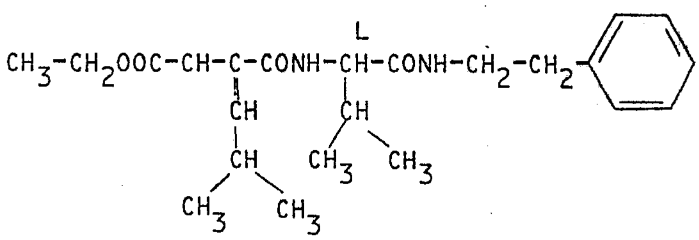

En partant de 4 g (11,9 mM) du composé précédent (22) et de 2,39 g (11,9 mM)d'acide carbéthox'-3-isobutylidène-2-propanoïque et en appliquant les conditions de traitement décrites dans l'obtention du composé (17), on obtient un produit solide brut qui, après lavage par une solution de 100 cm$^3$ d'éther et 50 cm$^3$ d'éther de pétrole, donne 4,77 g (11,9 mM) du composé (23), N-phénéthyl-Nα(éthoxycarbonyl-3-isobutylidène-2-propanoyl)-L-valinamide sous la forme d'un gel blanchâtre.
Rf = 0,48 Silicagel, acétate d'éthyle-cyclohexane (50/50 en volumes).
Rendement = 100 %.

(24) N-phénéthyl-Nα-(carboxy-3-isobutylidène-2-propanoyl)-L-valinamide

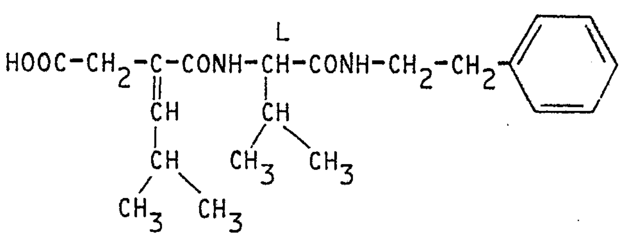

A la solution refroidie vers 10°C de 4,75 g (11,8 mM) du composé (23) dans 40 cm$^3$ d'éthanol, sont introduits lentement en agitant 13 cm$^3$ d'hydroxyde de sodium 1N.

Après 30 min d'agitation à 10° C et 2 h à une température voisine de 20° C, la solution réactionnelle, traitée dans les conditions d'obtention du dérivé (18), donne 3,63 g (9,7 mM) du composé (24), N-phénéthyl-Nα-(carboxy-3-isobutylidène-2-propanoyl)-L-valinamide, sous la forme d'une meringue blanche.
Rf = 0,35 Silicagel, chloroforme-méthanol (90/10 en volumes).
Rendement = 82 %.

(25) N-phénéthyl-Nα-(N-benzyloxycarbamoyl-3-isobutylidène-2-propanoyl)-L-valinamide

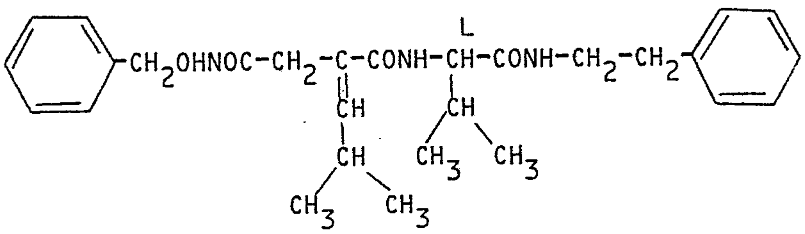

A une solution de 3,0 g (8,1 mM) du composé (24) dans 70 $cm^3$ de diméthylformamide, sont ajoutés successivement en agitant :
- une solution de 1,29 g (8,1 mM) de chlorhydrate de O-benzyl-hydroxylamine dans 50 $cm^3$ de diméthylformamide,
- 1,13 $cm^3$ (8,1 mM) de triéthylamine,
- 1,24 g (8,1 mM) d'hydroxybenzotriazole hydrate.

Le mélange refroidi à 10° C est additionné en 2 min d'une solution de 3,46 g (8,16 mM) de p-toluènesulfonate de [(méthyl-4-morpholino)-2-éthyl]-3-cyclohexyl-1-carbodiimide dans 30 $cm^3$ de diméthylformamide.

Après 1 h d'agitation à 10° C, puis 24 h à une température voisine de 20° C, le mélange réactionnel est concentré à sec sous pression réduite (1 mm de mercure) à 50° C.

Le résidu est dissous dans 300 $cm^3$ de chloroforme.

La solution obtenue est lavée dans les conditions décrites pour l'obtention du composé (19) puis concentrée sec sous pression réduite (20 mm de mercure) à 50° C.

Le solide obtenu, trituré dans 50 $cm^3$ d'éther, séparé par filtration, puis cristallisé dans 40 $cm^3$ d'acétonitrile, conduit à 1,1 g (2,29 mM) du composé (25), N-phénéthyl-Nα-(N-benzyloxycarbamoyl-3-isobutylidène-2-propanoyl)-L-valinamide, sous la forme d'un solide blanc.
Rf = 0,8 Silicagel, chloroforme-méthanol (90/10 en volumes).
Point de fusion (Kofler) = 182° C.
Rendement = 28 %.

(26) N-pénéthyl-Nα-(N-hydroxy-isobutyl-2-succinamoyl)-L-valinamide forme B

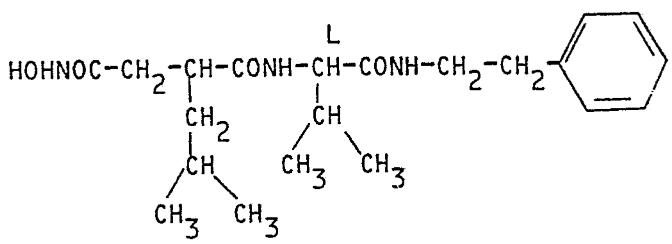

A une suspension de 1,1 g de palladium sur noir (à 10 % de palladium) dans 15 $cm^3$ de méthanol et 3 $cm^3$ d'acide acétique, préalablement saturée en hydrogéne, est ajoutée une solution de 1,1 g (2,29 mM) du composé (25) dans 45 $cm^3$ de méthanol et 2 $cm^3$ d'acide acétique.

Après agitation pendant 4 h à pression ordinaire, la suspension réactionnelle est purgée pendant 30 min par un courant d'azote.

Le catalyseur est éliminé par filtration, le filtrat est concentré à sec sous pression réduite (20 mm de mercure, puis 1 mm de mercure) à 40° C.

Le résidu, trituré dans 100 $cm^3$ d'éther, donne une poudre qui est séparée par filtration puis solubilisée dans 20 $cm^3$ d'un mélange chloroforme-méthanol (96/4 en volumes).

La solution est chargée sur une colonne de 3,4 cm de diamétre contenant 200 g de silice neutre (0,04-0,063 mm) puis est éluée avec 2,5 1 de mélange chloroforme-méthanol (96/4 en volumes) en recueillant des fractions de 20 $cm^3$.

Les fractions 107 à 125 réunies, sont concentrées à Le résidu, trituré dans l'éther, séparé par filtration,

séché sous pression réduite (20 mm de mercure) à 20°C, donne 57 mg (0,15 mM) du composé (26), N-phénéthyl-Nα-(N-hydroxy-isobutyl-2-succinamoyl)-L-valinamide, forme B, sous la forme d'un solide beige clair.

Rf = 0,38, Silicagel, chloroforme-méthanol (90/10 en volumes).

Point de fusion (Kofler) = 192°C.

Spectre de RMN DMSO $d_6$ (250 MHz, δ en ppm, J en Hz)

10.4, bs, 1H, NH ou OH

8.7, bs, 1H ou OH

7.95, t, 1H, $NHCH_2$

7.80, d, J = 8,5 1H, NHCH

7.15, à 7.35, m, 5H, $C_6H_5$

4.10, dd, (J = 8,5 et 8,5), 1H, CHα

3.30, m, 2H, $CH_2NH$

2.80, m, 1H, CHCO

2.70, t, 2H, $CH_2$-$C_6H_5$

2 et 2.15, ABX, 2H, $CH_2CO$

1.9, m, 1H, CH isopropyle

1.45, m, 1H, CH isopropyle

1.05 et 1.45, m. 2H, $CH_2$

0.80, m, 4 ⁄ 3H, 4 $CH_3$

Exemple 6

N-benzyl-Nα-(N-hydroxy-isobutyl-2-succinamoyl)-L-Norleucinamide

(27) Acide N-benzyloxycarbamoyl-3-isobutylidène-2-propanoïque

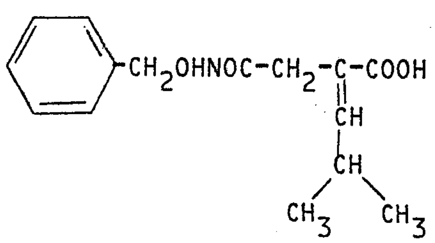

A une solution de 1,1 g (7,38 mM) d'anhydride isobutylidène succinique dissous dans 8 $cm^3$ de toluène sec, est ajoutée une solution de chlorhydrate de O-benzylhydroxylamine (1,41 g 8,86 mM) dans 10 $cm^3$ de toluène sec et de 1,24 $cm^3$ de triméthylamine (8,86 mM). Après 10 min d'agitation, le précipité formé est essoré et la phase organique est extraite par l'eau et la soude N. Les solutions aqueuses alcalines réunies (pH 9) sont acidifiées par HCl N jusqu'à pH 2-1 puis extraites par l'éther (2 × 25 $cm^3$). Les phases éthérées réunies sont lavées par l'eau (25 $cm^3$), séchées sur sulfate de sodium, cristallisées, filtrées puis concentrées à sec sous pression réduite pour donner le produit attendu sous forme d'huile (1,22 g ; 60 %) :

Rf = 0,54 (chloroforme/méthanol/acide acétique 9/1/0,1).

(28) N-benzyl-Nα-tertiobutyloxycarbonyl-L-norleucinamide

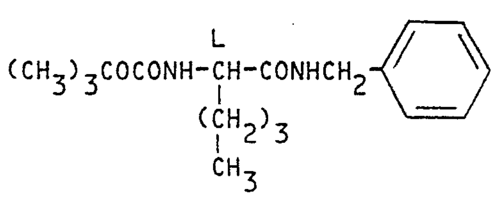

3,8 g (16,43 mM) de tertiobutyloxycarbonyle-norleucine dans 15 $cm^3$ de diméthylformamide (DMF) et 1,76 g (16,43 mM) de benzylamine sont condensés en présence de 2,52 g (16,43 mM) d'hydroxybenzotriazole dans 15 $cm^3$ de DMF et de 3,73 g (18,07 mM) de dicyclohexylcarbodiimide dans 15 $cm^3$ de DMF. Après traitement dans les conditions d'obtention du composé (5), on obtient une huile épaisse (5,45 g ; 104 %). Cette huile est traitée par flash chromatographie : diamétre de la colonne = 5 cm : hauteur de silice = 15 cm ; éluant = éther/cyclohexane 5/5 ; fractions recueillies = 60 $cm^3$ Les fractions de 7 à 17 sont rassemblées,

concentrées à sec sous pression réduite pour conduire au composé attendu sous forme d'huile épaisse (5,09 g ; 97 %) ;
Rf = 0,33 (éther/cyclohexane 5/5).

(29) N-benzyl-L-norleucinamide

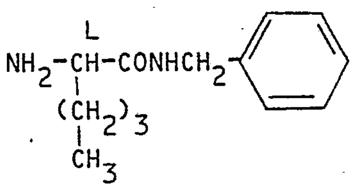

5,08 g (15,85 mM) du composé (28) sont agités 30 min 0°C puis 30 min à température ambiante en présence de 23 $cm^3$ d'acide trifluoroacétique (1,5 $cm^3$/mM). Après traitement dans les conditions de préparation du composé (10), on obtient le produit attendu sous forme d'une mousse (4,23 g ; 80 %) ;
Rf = 0,2 (chloroforme/méthanol/acide acétique).

(30) N-benzyl-Nα-(N-benzyloxycarbamoyl-3-isobutylidène-2-propanoyl)-L-norleucinamide

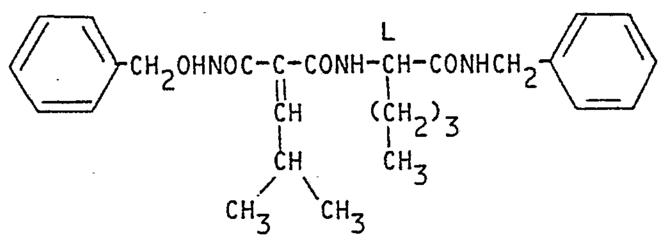

A une solution de 800 mg (2,28 mM) de l'acide (27) dans 5 $cm^3$ de diméthylformamide et de 0,381 $cm^3$ de triéthylamine refroidie à 0°C, sont ajoutées successivement :
- une solution de 1,09 g (3,25 mM) de trifluoroacétate de N-benzyl-L-norleucinamidë dans 10 $cm^3$ de diméthylformamide et 0,454 $cm^3$ de triéthylamine,
- une solution de 612 mg (2,97 mM) de dicyclohexylcarbodiimide dans 5 $cm^3$ de diméthylformamide.

Après 1 h d'agitation à 0°C puis environ 20 h à la température ambiante, le mélange réactionnel est traité dans les conditions d'obtention du composé (5). Le résidu obtenu, après précipitation dans le mélange éther/éther de pétrole (1/1), conduit au produit attendu sous forme d'une poudre blanche (900 mg ; 65 %) ;
Rf = 0,31 (chloroforme/méthanol/acide acétique 9/1/0,1).

(31) N-benzyl-Nα-(N-hydroxy-isobutyl-2-succinamoyl)-L-norleucina mide

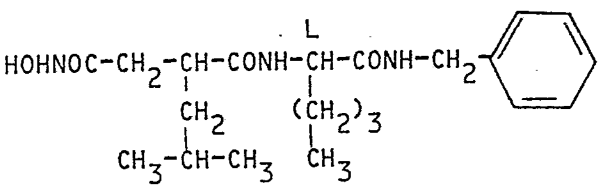

90 mg (0,19 mM) du composé (30) sont hydrogénés en présence de 38 mg de charbon palladié à 10 % (200 mg/mM) dans 2 $cm^3$ de méthanol et 0,100 $cm^3$ d'acide acétique. A la fin de la réaction, le mélange réactionnel est traité dans les conditions de préparation du composé (8) pour donner un solide pâteux. Ce solide est dissous dans 0,209 $cm^3$ de soude N (pH 6) et agité pendant 1 h. Un léger insoluble est alors éliminé et le filtrat concentré à sec sous pression réduite. Le résidu obtenu est repris par 15 $cm^3$ d'eau.
La solution aqueuse ainsi obtenue (pH 6) est extraite par 3 × 10 $cm^3$ d'acétate d'éthyle. Les phases organiques rassemblées sont séchées sur sulfate de sodium cristallisé, filtrées et concentrées à sec sous pression réduite pour donner le produit attendu (A) sous forme d'une mousse (23 mg ; 32 %) ; ce produit est constitué d'un mélange de 2 diastéréoisomères A + B ;

Rf confondus = 0,49 (chloroforme/méthanol/acide acétique 9/1/0,1) (270 MHz, δ en ppm)
RMN-DMSO-$d_6$ = pic large centré sur 10,36 ppm (1H, NHO) ; pic large centré sur 8,70 (1H,N-OH) ; 8,36 et 8,25 (1H, t, NH-$CH_2C_6H_5$) ;
8,25 et 7,91 (1H, d, NH-Nle) ; 7,18 (5H, m, $C_6H_5$-) massif centré sur 4,2 (3H, $CH_2$-$C_6H_5$ + Hα Nle) ; pic large centré sur 2,66 (1H,

$-H_2C-CH-)$ ; entre 2,25 et 1,89 (4H, m, $CO-H_2C-CH-CO)$ ;
       $CH_2-$                                                   $CH_2-$

1,73 et 1,58 (1H, massif, $CH-CH_3)$ ; entre 1,52 et 0,88 (9H, $-(CH_2)_3$
                                              $CH_3$

et $CH_3-CH_2)$ 0,77 (6H, $-CH-CH_3)$ ;
                                  $CH_3$

Exemple 7

(32) N-benzyl-Nα-benzyloxycarbonyl-Nε-tertiobutyloxycarbonyl-L-lysinamide

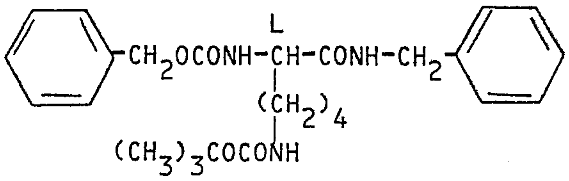

5 g (13,14 mM) de Nα-benzyloxycarbonyl-Nε-tertiobutyloxycarbonyl-L-lysine dans 20 $cm^3$ de DMF sec et 1,41 g (13,14 mM) de benzylamine sont condensés en présence de 2,01 g (13,14 mM) d'hydroxybenzotriazole dans 10 $cm^3$ de DMF sec et de 2,98 g (14,54 mM) de dicyclohexylcarbodiimide dans 10 $cm^3$ de DMF sec. Après traitement dans les conditions de préparation du composé (5), on obtient un solide pâteux. Ce solide est recouvert d'éther pour donner un précipité de N-benzyl-Nα-benzyloxycarbonyl-Nε-tertiobutyloxycarbonyl-L-lysinamide (5,51 g ; 89 %) ;
Rf = 0,72 (chloroforme/méthanol 9/1).
Point de fusion = 105° C.

(33) N-benzyl-Nα-tertiobutyloxycarbonyl-L-lysinamide

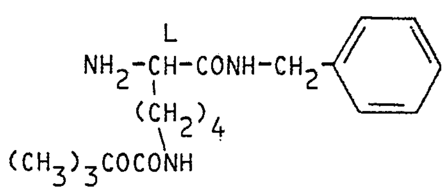

3 g (6,4 mM) du composé précédent (32) sont solubilisés dans 20 $cm^3$ de méthanol et ajoutés à une suspension de 384 mg de charbon palladié à 10 % (100 mg/mM) dans 5 $cm^3$ de méthanol, préalablement saturée d'hydrogéne. Après 4 h d'agitation à température ambiante, sous atmosphére d'hydrogène la pression ordinaire, le catalyseur est éliminé par filtration ; le filtrat est concentré à sec sous pression réduite pour donner le N-benzyl-Nα-tertiobutyloxycarbonyl-L-lysinamide sous forme d'une huile épaisse (2,15 g ; 100 %) ;
Rf = 0,37 (chloroforme/méthanol 9/1).

(34) N-benzyl-Nα-(éthoxycarbonyl-3 isobutylidène-2-propanoyl)-Nε-tertiobutyloxycarbonyl-L-lysinamide

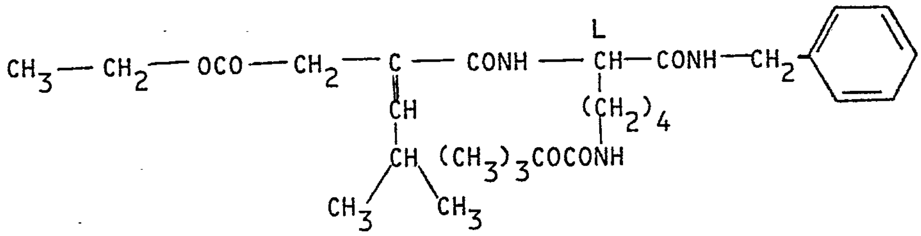

597 mg (2,98 mM) d'acide éthoxycarbonyl-3-isobutylidène-2-propanoïque dans 10 $cm^3$ de DMF et 1 g (2,98 mM) de N-benzyl-Nε-tertiobutyloxycarbonyl-L-lysinamide sont condensés en présence de 456 mg (2,98 mM) d'hydroxybenzotriazole dans 5 $cm^3$ de DMF et de 676 mg (3,28 mM) de dicyclohexylcarbodiimide dans 5 $cm^3$ de DMF.

Après traitement dans les conditions de préparation du composé (5), on obtient le produit attendu sous forme d'huile épaisse (1,50 g ; 100 %) ;
Rf = 0,71 (chloroforme/méthanol 9/1).

(35) N-benzyl-Nα-(carboxy-3-isobutylidène-2-propanoyl)-Nε-tertiobutyloxycarbonyl-L-lysinamide

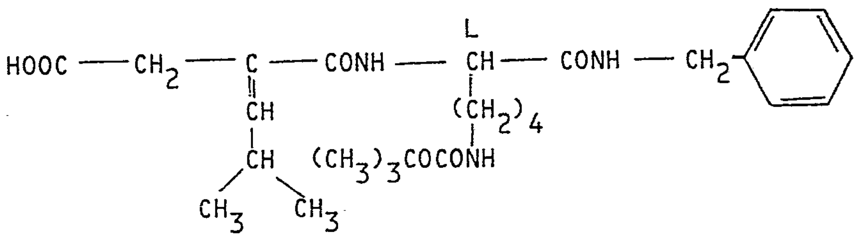

583 mg (1,13 mM) du composé précédent (34) dissous dans 5 $cm^3$ d'éthanol/eau 3/1 sont agités pendant 1 h à 0° C puis 2 h à température ambiante , en présence de 1/2 $cm^3$ de soude N. A la fin de la réaction, après traitement dans les conditions de préparation du composé (2), on obtient le produit attendu sous forme de mousse (4,59 mg ; 83%) ;
Rf = 0,47 (chloroforme/méthanol/acide acétique 9/1/0,1).

(36) N-benzyl-Nα-(N-benzyloxycarbamoyl-3-isobutylidène-2 propanoyl-Nε-tertiobutyloxycarbonyl-L-lysinamide

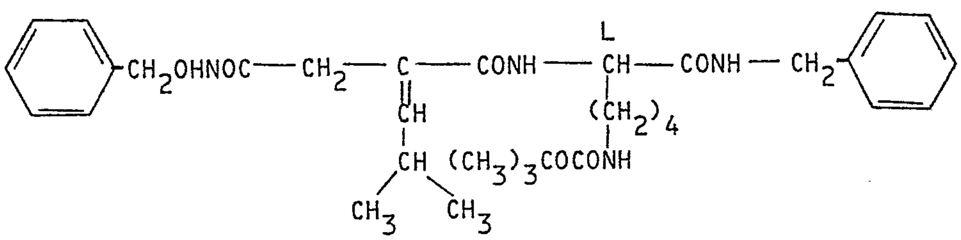

445 mg (0,91 mM) du composé précédent (35) dans 10 $cm^3$ de DMF et 145 mg (0,91 mM) de chlorhydrate de O-benzylhydroxylamine dans 3 $cm^3$ de DMF et 0,128 $cm^3$ de triéthylamine sont condensés en présence de 139 mg (0,91 mM) d'hydroxybenzotriazole dans 5 $cm^3$ de DMF et de 207 mg (1 mM) de dicyclohexylcarbodiimide dans 2 $cm^3$ de DMF. Après traitement dans les conditions de préparation du composé (5), on obtient le produit attendu sous forme d'une huile épaisse (447 mg ; 83 %) ;
Rf = 0,65 (chloroforme/méthanol 9/1)

(37) N-benzyl-Nα-(N-hydroxyisobutyl-2-succinamoyl)-Nε-tertiobutyloxy carbonyl-L-lysinamide

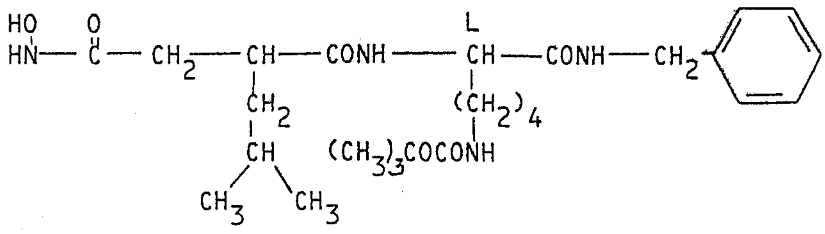

A une suspension de 16 mg de charbon palladié à 10 % dans 2 $cm^3$ de mélange méthanol/acide acétique/eau (4/5/1) préalablement saturée d'hydrogéne, sont ajoutés 60 mg (0,1 mM) du composé précédent (36) dans 1 $cm^3$ de méthanol. Après 3 h d'agitation à température ambiante, sous atmosphère d'hydrogène et traitement dans les conditions de préparation du composé (8), on obtient le dérivé attendu sous forme d'une huile épaisse (51 mg ; 100 %) ;
Rf = 0,28 (chloroforme/méthanol 9/1).

(38) N-benzyl-Nα-(N-hydroxy-isobutyl-2-succinamoyl)-L-lysinamide, trifluoroacétate

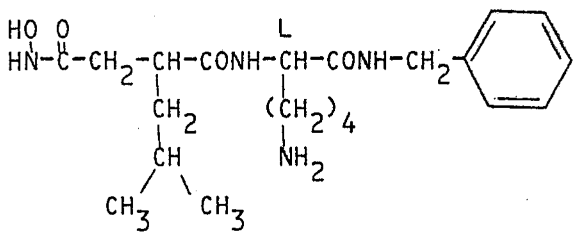 . $CF_3COOH$

50 mg (0,1 mM) du composé précédent (37) sont agités 30 min à 0° C puis 1 h à température ambiante en présence de 0,150 $cm^3$ d'acide trifluoroacétique. Après traitement dans les conditions de préparation du composé (10), on obtient les produits attendus sous forme de mousse (41 mg ; 79 %) ; cette mousse est constituée de 2 diastéréoisomères.
Rf confondus = 0,49 (chloroforme/méthanol 9/1).

RMN DMSO-$d_6$ (270 MHz, δ en ppm) = pic large centré sur 10,45 (1H, NH-O) ; 8,7 (1H, large, N-OH) ; 8,37 et 8,13 (1H, t, N$\underline{H}$-$CH_2$-$C_6H_5$) ; 8,22 et 7,9 (1H, d, NHα-lys) ; pic large centré sur 7,62 (3H, $NH_3$), 7,16 (5H, m, $C_6H_5$) ; massif centré sur 4,2 (3H, $CH_2$-$C_6H_5$ + Hα-lys); 2,65 (2H, pic large, -$CH_2$-N-lys) ; entre 2,35 et 1,88 (5H,CO-$H_2$C-CH-CO) ; 1,73 et 1,58 (1H, m, —$\underline{CH}$— $H_3C$ $CH_3$) entre 1,55 et 0,91 (6H, $CH_2$ -CH-($\underline{CH_2}$)$_2$-$CH_2$-N- + $\underline{CH_2}$-ipr) ; 0,79 (6H, m, CH— $\underline{H_3}C$ $\underline{CH_3}$)

Exemple 8

N-benzyl-Nα(N-hydroxy isobutyl-2-succinamoyl)-L-méthionine amide (39) N-benzyl-Nα -tertiobutyloxycarbonyl-L-méthionine amide

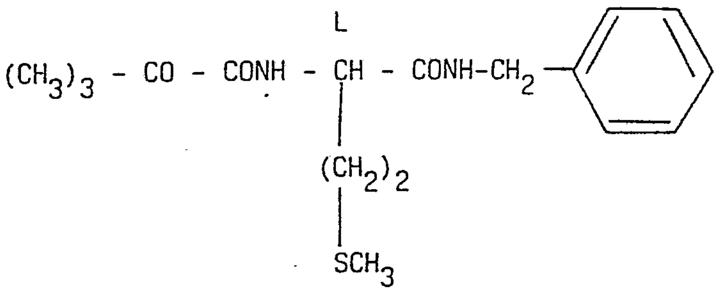

5,01 g (20,1 mM) de tertiobutyloxycarbonyle L-méthionine dans 15 $cm^3$ de diméthylformamide (DMF) et 2,15 g (20,1 mM) de benzylamine sont condensés en présence de 3,08 g (20,1 mM) d'hydroxybenzotriazole dans 6 $cm^3$ de DMF et de 4,56 g (22,1 mM) de dicyclohexylcarbodiimide dans 10 $cm^3$ de DMF.
Après traitement dans les conditions d'obtention du composé (5), on obtient, après précipitation par l'éther, un solide (6,8 g; 100 %) Rf = 0,79 (chloroforme/méthanol 9/1) ; F = 90°C.

(40) N-benzyl-L-méthionine amide

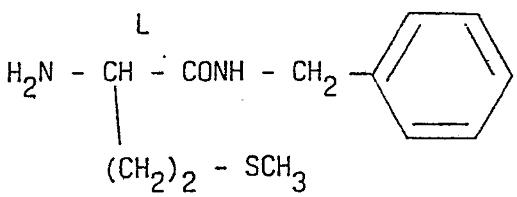

2 g (5,91 mM) du composé précédent (39) sont traités dans les conditions d'obtention du composé (29) pour donner le produit attendu, sous forme d'aiguilles blanches (2,07 g ; 99 %) ;
Rf = 0,29 (chloroforme/méthanol/acide acétique 9/1/0,1).

(41) Acide N-tertiobutyloxycarbamoyl-3-isobutylidène-2-propanoïque

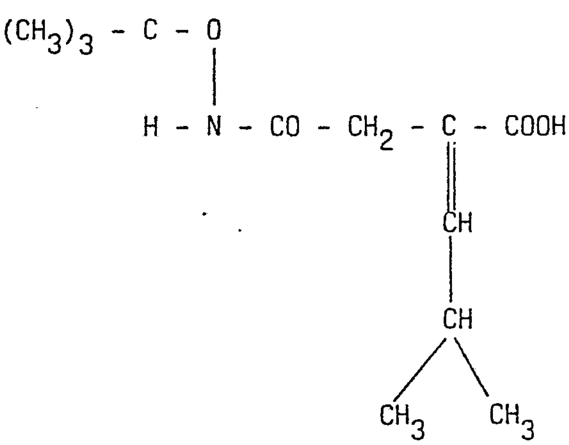

On traite, dans les conditions de préparation du composé (27) 1,1 g (7,38 mM) d'anhydride d'acide isobutylène succinique dans 8 $cm^3$ de toluène ec ; 1,11 g (8,86 mM) de chlorhydrate de O-tertiobutylhydroxylamine dans 10 $cm^3$ de toluène sec et 1,24 $cm^3$ (8,86 mM) de triéthylamine pour obtenir le produit attendu sous forme de solide blanc (1,03 g ; 57 %) Rf = 0,21 (chloroforme, méthanol 9/1).

(42) Acide-N-tertiobutyloxycarbamoyl-3-isobutyl-2 propanoïque

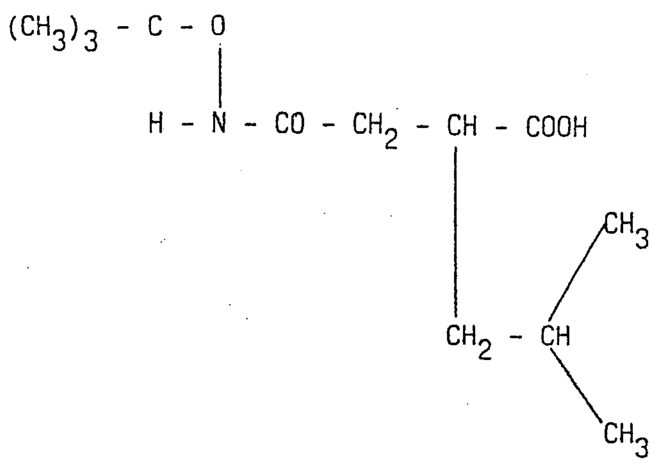

500 mg (2,05 mM) du composé précédent (41) dissous dans 5 $cm^3$ de méthanol sont hydrogénés en présence d'une suspension de 200 mg de charbon palladié (10 %) dans 5 $cm^3$ de méthanol, dans les conditions d'obtention du composé (8) pour donner le produit attendu sous forme de poudre cristalline blanche (500 mg ; 99 %) ; Rf = 0,26 (chloroforme/méthanol 9/1) ; F = 157°C.

(43) N-benzyl-Nα -(N-tertiobutyloxycarbamoyl 3 isobutyl-2-propanoyl)-L-méthionine àmide

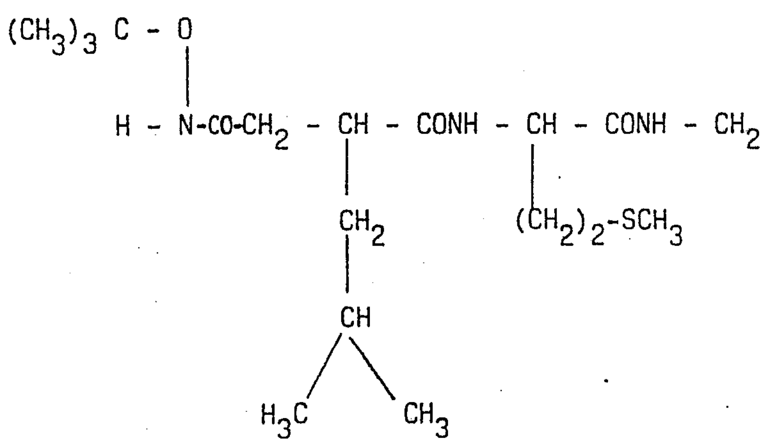

200 mg (0,81 mM) de l'acide précédent (42) dans 5 $cm^3$ de chloroforme et 0,107 $cm^3$ de triéthylamine sont condensés avec 2,19 mg (0,92 mM) de N-benzyl-L-méthionine amide dans 10 $cm^3$ de chloroforme, en présence de 172 mg (0,83 mM) de dicyclohexyl carbodiimide dans les conditions de préparation du composé (30) pour donner le produit attendu sous forme d'une huile épaisse (322 mg ; 85 %) ; Rf = 0,58 (chloroforme/méthanol 9/1).

(44) N-benzyl-Nα-(N-hydroxy isobutyl-2 succinamoyl)-L-méthionine amide

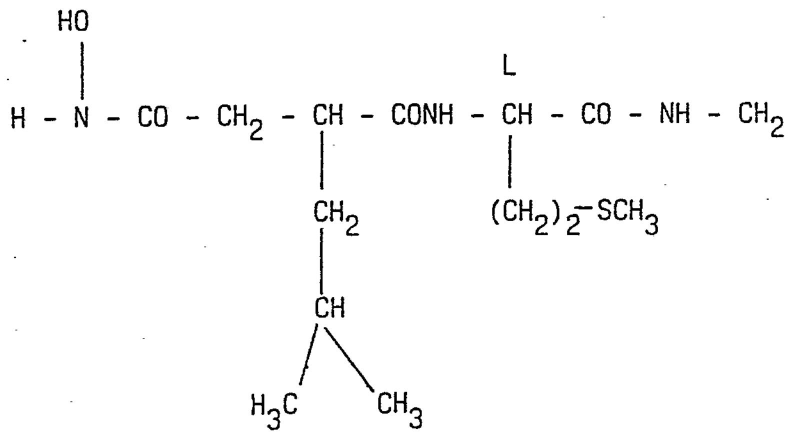

320 mg (0,69 mM) du composé précédent (43) sont agités à 0°C en présence de 2 $cm^3$ d'acide trifluoracétique et 2 $cm^3$ de trifluoracétate de bore pendant 30 minutes. Le mélange est alors concentré à sec sous pression réduite pour donner un résidu qui est repris par l'éther pour conduire à un mélange de diastéréoisoméres (290 mg).
280 mg du mélange sont traités en flash chromatographie avec une colonne de 3 cm en recueillant des fractions de 20 $cm^3$ et avec comme éluant un gradient de chloroforme/méthanol 9/1 puis 7/3 pour éliminer les impuretés de tête. Les deux diastéréoisomères sont élués dans les fractions 51 à 55 à l'aide du mélange méthanol/acide acétique 10/0,25 (90 mg ; 32 %). 50 mg du produit ainsi élué sont séparés sur colone de silice (diamétre 1,2 cm ; hauteur de silice : 60 cm ; éluant : chloroforme/méthanol/acide acétique 10/0,5/0,1 ; fractions de 350 gouttes ; débit : 1 goutte toutes les 4 secondes) pour donner les deux isoméres : 22 mg (fractions 28 à 39) et 15 mg (fractions 40 à 75) ; Rf = 0,30 et 0,19 (chloroforme/méthanol/acide acétique 10/0,5/0,1).
La voie de synthèse qui vient d'être donnée est spécifique lorsque W est un reste de méthionine.

L'activité anticollagénasique des composés selon la présente intention a été déterminée sur de la collagénase synoviale d'origine porcine ou humaine isolée des cellules synoviales en culture selon le mode opératoire décrit par Cawston et Barrett (Cawston et Barrett (1979), Anal. Biochem. 99, 340-345). La collagénase a été incubée à 37°C avec du collagène radiomarqué et son activité a été déterminée par la libération des peptides solubles produits par la dégradation enzymatique du collagéne. La concentration inhibitrice $CI_{50}$ pour chaque composé a été déterminée par mesure de l'activité de l'enzyme en présence de l'inhibiteur à une concentration entre $10^{-5}$ et $10^{-8}$ M.

Des résultats représentatifs de ces essais sont donnés pour les composés selon l'inténtion dans le tableau I.

L'activité anticollagénasique a été également déterminée dans un modéle de résorption osseuse décrite par Delaissé et al (Delaissé et al (1985), Biochem. Biophys. Res. commun., 133, 483-490). Ce modéle, qui utilise la dégradation de cartilage et d'os dans un organe isolé, est plus physiologique que le dosage direct de l'activité enzymatique in vitro. les résultats des essais sont groupés dans le tableau II ; les mêmes inhibiteurs se révélent actifs dans les deux systémes.

La spécificité de l'inhibition a été mesurée par étude de plusieurs autres enzymes y compris des métalloprotéases autres que la collagénase. Les inhibiteurs selon l'intention ont montré une activité considérable vis-à-vis de la collagénase.

La stabilité des composés selon l'intention à l'influence des enzymes protéolytiques a été également étudiée. Les inhibiteurs les plus puissants se sont montrés résistants aux protéases suivantes : amino peptidase M, thermolysine, carboxy peptidase A et B, chymotrypsine, trypsine, thrombine, ACE, papaïne, pepsine, kallikréine, jus gastrique et intestinal.

La toxicité des composés a été évaluée in vitro sur les cellules humaines en culture et chez l'animal. Aucune cytotoxicité significative n'a été observée et aucune toxicité n'a été observée chez la souris à la dose de 0,5 g/kg par voie sous-cutanée.

Ainsi, les composés selon la présente invention sont utiles comme médicaments dans le traitement des maladies impliquant une destruction excessive du collagène par la collagénase. Comme il a été mentionné dans l'introduction, ces maladies incluent les arthrites, l'arthrose, la maladie périodontale, les ulcérations, d'autres maladies avec destruction du tissu conjonctif et, peutêtre, l'invasion tumorale et la résorption osseuse.

Le mode d'administration des inhibiteurs de collagénase selon l'intention dépend de la maladie à traiter et peut se faire par administration locale (ulcération, maladie périodontale) ou systémique (entérale ou parentérale) pour les autres indications. Par exemple, dans le traitement d'une arthrite, le composé peut être administré par voie orale, intraveineuse, sous-cutanée ou intramusculaire ou, éventuéllement, directement dans les tissus concernés par injection intra-articulaire. Les composés peuvent être utilisés en solution

aqueuse, ou sous la forme d'une pommade ou d'un gel ou d'une formulation similaire pour les applications locales, ou éventuellement, sous une forme galénique permettant une libération lente du produit par exemple par encapsulation dans un polymére inerte.

La dose du composé dépend du mode d'administration, de la formulation sélectionnée et de la condition du sujet sous traitement et peut être comprise entre 100 µg et 10 mg/kg en dose unique ou répétée.

TABLEAU I

ACTIVITE ANTICOLLAGENIQUE

| Composé | % inhibition | Concentration M (mole) |
|---|---|---|
| Exemple 1 | 50 | $3{,}2 \times 10^{-7}$ |
| Exemples 2 et 3 (isomère B) | 50 | $3{,}5 \times 10^{-8}$ |
| Exemple 4 | 50 | $8 \times 10^{-8}$ |
| Exemple 5 | 50 | $7{,}5 \times 10^{-8}$ |

TABLEAU II

| | % protection résorption osseuse (libération $Ca^{++}$) | inhibition collagénase $IC_{50}$ (µM) |
|---|---|---|
| Inhibition (diastéréoisomères) | | |
| Exemple 2, isomère B 8 µM | 90 - 100 | 0,035 |
| Exemple 2, isomère B 25 µm | 90 - 100 | |
| Exemple 2, isomère A 8 µm | 0 | 16 |
| Exemple 2, isomère A 25 µm | 0 | |

TABLEAU III

SPECIFICITE DE L'INHIBITION

| ENZYME | INHIBITEUR (% inhibition) | | | |
| --- | --- | --- | --- | --- |
| | Composé de l'ex.1 | | Composé de l'ex.2 mélange des isomères A et B | |
| | $10^{-5}$M | $10^{-7}$M | $10^{-5}$M | $10^{-7}$M |
| Collagénase porcine | 100 | 23 | 100 | 52 |
| Collagénase humaine | 100 | 12 | 100 | 30 |
| ACE | 4 | 0 | 40 | 4 |
| Carboxypeptidase A | 0 | 0 | 0 | 0 |
| Carboxypeptidase B | 0 | 0 | 0 | 0 |
| α-chymotrypsine | 0 | 0 | 0 | 0 |
| Plasmine | 0 | 0 | 0 | 0 |
| Thrombine | 0 | 0 | 5 | 0 |
| Trypsine | 0 | 0 | 0 | 0 |
| Papaïne | 5 | 0 | 5 | 0 |
| Pepsine | 19 | 0 | 19 | 0 |

**Revendications**

1. Composés caractérisés en ce qu'ils répondent à la formule générale :

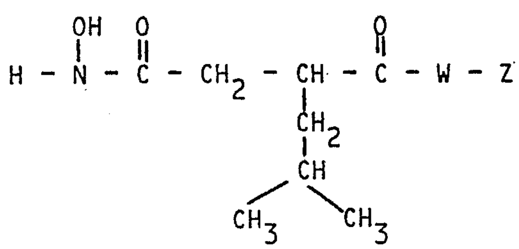

(I)

dans laquelle
- W représente un reste d'acide aminé choisi parmi la valine, la lysine, la norleucine et la méthionine ; et
- Z représente un radical amino ou alcoylamino dont la partie alcoyle qui contient 1 ou 2 atomes de carbone est substituée par un radical phényle ou trifluorophényle,

ainsi que leurs diastéréoisoméres et leurs sels d'addition avec les acides pharmaceutiquement acceptables.

2. Composés selon la revendication 1, caractérisés en ce qu'ils répondent à la formule I dans laquelle W représente un groupe L-valyle.

3. Composés selon la revendication 1, choisis parmi les suivants :
- N α -(N-hydroxy isobutyl-2 succinamoyl) L valinamide
- N Benzyl Nα -(N-hydroxy isobutyl-2 succinamoyl) L valinamide
- N (trifluorométhyl-4 benzyl) Nα -(N-hydroxy isobutyl-2 succinamoyl) L valinamide
- N -phénéthyl Nα -(N-hydroxy isobutyl-2 succinamoyl) L valinamide
- N Benzyl Nα -(N-hydroxy isobutyl-2 succinamoyl) L norleucine
- N Benzyl Nα -(N-hydroxy isobutyl-2 succinamoyl) L-lysinamide.

4. Procédé de préparation des composés répondant à la formule générale :

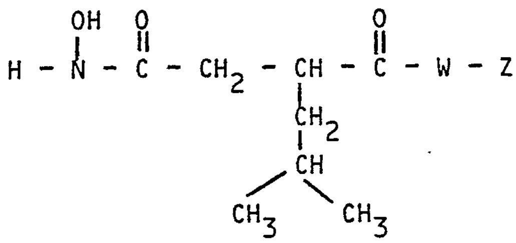

(I)

dans laquelle
- W représente un reste d'acide aminé choisi parmi la valine, la lysine, la norleucine et la méthionine ; et
- Z représente un radical amino ou alcoylamino dont la partie alcoyle qui contient 1 ou 2 atomes de carbone est substituée par un radical phényle ou trifluorophényle, caractérisé en ce qu'il comprend les étapes :

1)

a) la réaction de condensation d'un ester d'acide de formule :

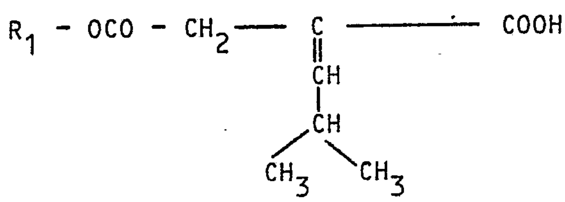

dans laquelle $R_1$ désigne un radical alkyle de 1 à 6 atomes, avec un dérivé d'aminoacide de formule

H - W - Z

dans laquelle W et Z sont définis comme ci-dessus, pour former le composé de formule

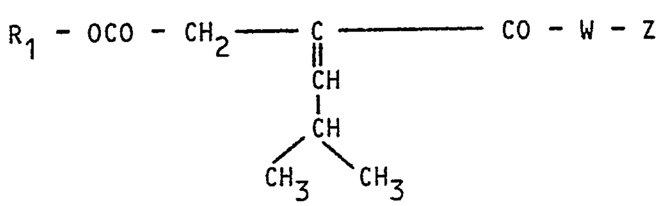

b) l'hydrolyse en milieu alcalin de l'ester formé pour obtenir l'acide correspondant,

c) la condensation de l'acide obtenu avec la O-benzylhydroxylamine pour former le dérivé

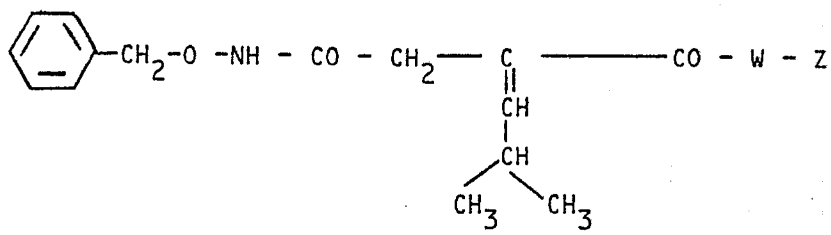

ou bien

a') la réaction de condensation d'un anhydride de formule

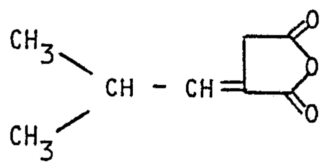

avec la O-benzylhydroxylamine, pour former l'acide de formule

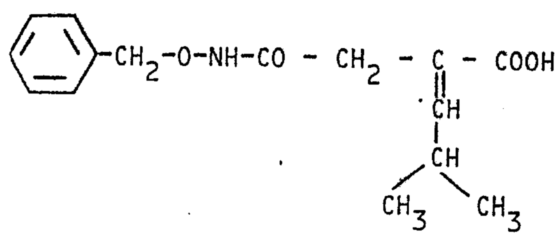

b') la condensation de l'acide obtenu avec un dérivé d'aminoacide de formule générale

H - W - Z

dans laquelle W et Z sont définis comme ci-dessus pour former le dérivé

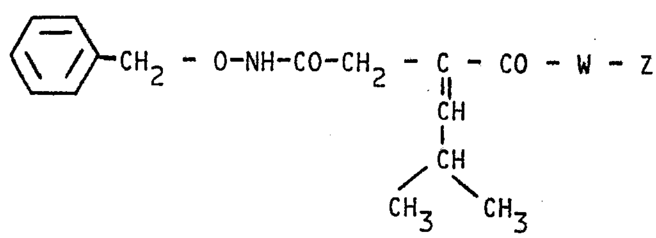

suivies de :

2) l'hydrogénation catalytique du produit obtenu pour obtenir le composé désiré de formule I,

ou bien

1') la réaction de condensation d'un anhydride de formule

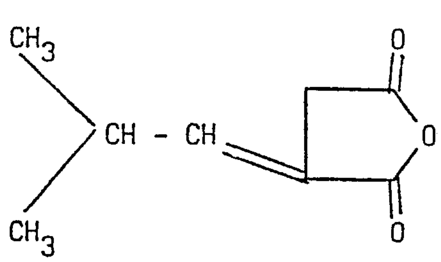

avec la O-tertiobutyloxyhydroxylamine, pour former l'acide N-tertiobutyloxycarbamoyl-3-isobutylidène-2-propanoique de formule

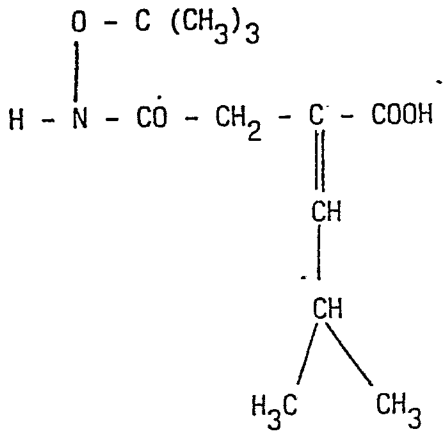

2') l'hydrogénation catalytique de l'acide obtenu en 1') pour former l'acide N-tertiobutyloxycarbamoyl 3-isobutyl-2-propanoïque de formule :

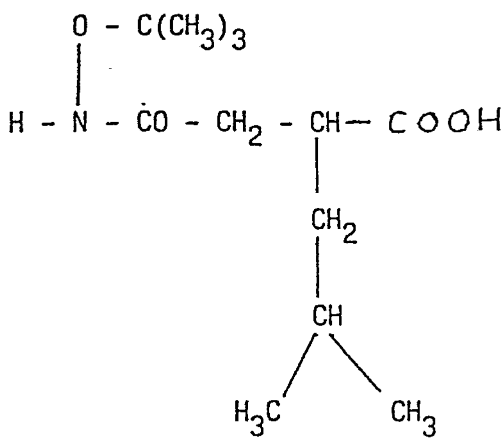

3') la condensation de l'acide obtenu en 2') avec un dérivé d'amino acide de formule générale

H - W - Z

dans laquelle W et Z sont définis comme ci-dessus pour former le dérivé :

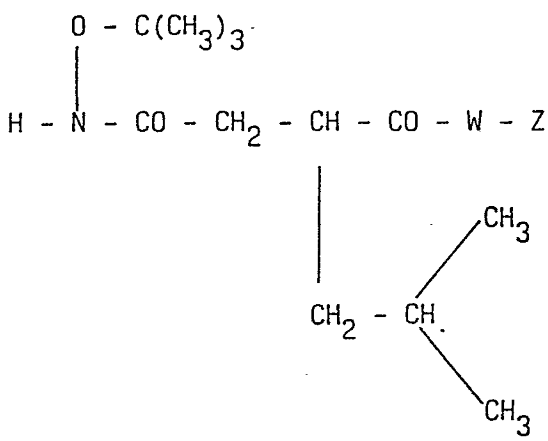

4') La réaction du composé ainsi formé avec un mélange acide trifluoroacétique/trifluoroacétate de bore, pour obtenir le composé désiré de formule I.

5. Composition pharmaceutique, caractérisée en ce qu'elle contient à titre d'ingrédient actif au moins un composé selon l'une des revendications 1 à 3, en association avec un véhicule ou support non toxique, pharmaceutiquement acceptable.

6. Composition pharmaceutique à activité d'inhibiteur de collagénase, caractérisée en ce qu'elle contient à titre d'ingrédient actif au moins un composé selon l'une des revendications 1 à 3, en association avec un véhicule ou support non toxique, pharmaceutiquement acceptable.

7. Composition pharmaceutique selon la revendication 6, caractérisée en ce qu'elle contient, à titre d'ingrédient actif au moins un composé selon l'une des revendications 1 à 3 sous la forme isomérique la plus active, déterminée après séparation.

8. Composition pharmaceutique selon la revendication 7, caractérisée en ce qu'elle contient, à titre d'ingrédient actif au moins un composé selon la revendication 2, sous la forme isomérique la plus active, caractérisée en RMN par le déplacement chimique situé à 2 ppm ou en dessous et correspondant au $> CH_{\beta}$ de la valine.

9. Utilisation des composés selon l'une des revendications 1 à 4 pour la fabrication d'un médicament destiné au traitement des maladies impliquant une destruction excessive du collagène par la collagénase.